## Europäisches Patentamt

## European Patent Office

## Office européen des brevets

(11) Veröffentlichungsnummer: **0 180 012**
A1

(12) # EUROPÄISCHE PATENTANMELDUNG

(21) Anmeldenummer: **85111361.3**

(22) Anmeldetag: **09.09.85**

(51) Int. Cl.⁴: **C 12 N 15/00**, C 07 H 21/04,
C 07 K 15/00, C 12 P 21/00,
C 12 N 1/20, A 61 K 39/29,
A 61 K 39/42, C 12 N 5/00,
G 01 N 33/576
// C12R1:19

(30) Priorität: **27.10.84 DE 3439400**

(43) Veröffentlichungstag der Anmeldung: **07.05.86**
**Patentblatt 86/19**

(84) Benannte Vertragsstaaten: **AT BE CH DE FR GB IT LI LU NL SE**

(71) Anmelder: **Asta-Werke Aktiengesellschaft Chemische Fabrik, Artur-Ladebeck-Strasse 128 - 152, D-4800 Bielefeld 14 (DE)**

(72) Erfinder: **Gerlich, Wolfram H., Prof. Dr., Schlagenweg 3e, D-3400 Göttingen (DE)**
Erfinder: **Heermann, Klaus-Hinrich, Dr., Max-Born-Ring 47, D-3400 Göttingen (DE)**
Erfinder: **Köchel, Heinrich G., Dr. rer. nat., Oeltzenstrasse 8, D-3000 Hannover 1 (DE)**
Erfinder: **Uy, Angela, Dipl.-Biol., Georg-Dehlio-Weg 10, D-3400 Göttingen (DE)**
Erfinder: **Thomssen, Reiner, Prof. Dr., Wilhelm-Weber-Strasse 29, D-3400 Göttingen (DE)**

(54) **Immunogene Polypeptidsequenz des Hepatitis B-Virus.**

(57) Immunogene Peptidsequenz des Hepatitis B-Virus (Prä-S(1)-Sequenz) und Verfahren zur Herstellung solcher Peptide; monoklonaler Antikörper, der Hepatitis B-Virus-Peptidstrukturen neutralisiert.

ASTA-Werke Aktiengesellschaft, Chemische Fabrik
Artur-Ladebeck-Straße 128-152, 4800 Bielefeld 14


Immunogene Polypeptidsequenz des Hepatitis B-Virus


In der europäischen Patentanmeldung 0 020 251 wird ein Verfahren beschrieben, das Genom der DNA des Hepatitis B-Virus oder Fragmente dieses Genoms mittels Transfer-Vektoren in geeignete Wirtsorganismen einzuführen, so daß diese transformierten Mikroorganismen befähigt sind, Polypeptide zu produzieren, die von dem Genom des Hepatitis B-Virus beziehungsweise dessen Fragmenten kodiert werden. Nach diesem Verfahren wird beispielsweise ein Protein aus 226 Aminosäuren mit antigenen Eigenschaften (S-Protein) erhalten, welches zur Herstellung eines Impfstoffes gegen Hepatitis B-Virus-Erkrankungen verwendet werden kann.

Es wurde nun überraschend gefunden, daß Hepatitis B-Viren in ihrer Hülle eine stark immunogene Polypeptidsequenz von 108 oder 119 Aminosäuren enthalten, die durch einen bestimmten Genomausschnitt des Hepatitis Virus, nämlich der Prä-S(1)-Nucleotidsequenz, kodiert wird. Polypeptide, die mindestens eine Aminosäuresequenz enthalten, die dieser Prä-S(1)-Nucleotidsequenz entspricht (Prä-S(1)-Aminosäuresequenz) einschließlich der äquivalenten Varianten dieser Prä-S(1)-Aminosäuresequenz, sind zur Herstellung eines Impfstoffes gegen Hepatitis B-Virusinfektionen geeignet. Solche Peptide können beispielsweise auf gentechnologischem Wege hergestellt werden.

Im Gegensatz zu dem bekannten S-Protein aus der europäischen Patentanmeldung 0 020 251 läßt sich das

...

erfindungsgemäße Prä-S(1)-Protein ohne wesentliche Antigenitätsverluste in Bakterien herstellen oder beispielsweise auch aus dem Hepatitis B-Virus beziehungsweise dessen Hüllproteinen gewinnen.

Es hat sich gezeigt, daß in Abhängigkeit vom Gesundheitszustand, Geschlecht und Alter ein Teil der mit dem bekannten S-Protein geimpften Personen keine meßbaren Antikörper bildet, während bei anderen Personen der Antikörper wieder rasch verschwindet. Geimpfte Personen ohne Antikörper sind erfahrungsgemäß jedoch nicht geschützt.
Klinische Beobachtungen an Hepatitis B-Rekonvaleszenten zeigen andererseits, daß Antikörper gegen das S-Protein erst lange nach dem Verschwinden der Hepatitis B-Virus-Partikel im Serum erscheinen und viele der Rekonvaleszenten bilden trotz Ausheilung überhaupt keine Antikörper.

Aufgabe der Erfindung ist daher die Bereitstellung eines verbesserten Impfstoffes gegen Hepatitis B-Virus-Erkrankungen, dessen Wirkstoff vorzugsweise ein Polypeptid mit relativ niedrigem Molekulargewicht ist und wobei dieser Wirkstoff insbesondere in ausreichender und reiner Form hergestellt werden kann. Darüberhinaus ist eine Aufgabe der Erfindung die Gewinnung einer viel kleineren DNA-Sequenz (Prä-S(1)-Nucleotidsequenz) als die virale DNA selbst, welche eine Nucleotidsequenz enthält, die in der Lage ist, für eine solche kleinere Peptidsequenz mit immunogenen Eigenschaften zu kodieren (Prä-S(1)-Aminosäuresequenz). Diese Peptidsequenz kann, wenn sie in einen lebenden Wirtsorganismus eingebracht wird, die Erzeugung von Antikörpern durch diesen induzieren, welche fähig sind, den nämlichen Wirt letzt-

...

lich vor einer Infektion mit dem Virus der Virushepatitis B zu schützen.

Die Erfindung betrifft die durch die Patentansprüche definierten Gegenstände.
Die Abkürzungen für die Aminosäuren in den Ansprüchen sowie der Beschreibung sind die international üblichen Standardabkürzungen (siehe zum Beispiel Europa-Anmeldung 0 020 251, Seite 4).

Die überraschenden und nicht naheliegenden Vorteile der Erfindung liegen zum Beispiel in folgendem:

1.  Das erfindungsgemäße Prä-S(1)-Polypeptid induziert als Hepatitis B-Impfstoff Antikörper, die vorwiegend gegen das Hepatitis B-Virus und nur in geringem Maße gegen Hepatitis B-Oberflächen-Antigen-Partikel gerichtet sind. In Hepatitis B-Virushaltigen Proben befindet sich normalerweise ein rund tausendfacher Überschuß von nicht infektiösem Hepatitis Oberflächenantigen. Herkömmliche Hepatitis B-Impfstoffe induzieren Antikörper, die bei Vorliegen von nicht infektiösen Hepatitis B-Oberflächenantigenpartikeln mit verbraucht werden. Ein schützender anti-Prä-s(1)Antikörper kann dagegen in viel geringeren Mengen vorliegen als ein Antikörper, der aufgrund eines S-Protein-haltigen Impfstoffes gebildet wird, um die gleichen Hepatitis B-Virusmengen aus infektiösem Material abzubinden.

2.  Die Eigenschaften der Prä-S(1)-Aminosäuresequenz deuten darauf hin, daß sie das Hepatitis B-Virus-Antigen bildet, gegen das bei einer ausheilenden Hepatitis B-Virus-Infektion zuerst Antikörper

...

- 4 -

gebildet werden. Dies bedeutet eine verbesserte Immunogenität.

3. Antikörper gegen Peptide mit der Prä-S(1)-Aminosäuresequenz sind für den selektiven Nachweis von Hepatitis B-Viruspartikeln geeignet und als Immunadsorbens auch zur Entfernung von Hepatitis B-Viruspartikeln aus biologischen Flüssigkeiten. Antikörper gegen Peptide mit der Prä-S(1)-Aminosäuresequenz sind weiterhin in der Diagnostik und der Verlaufsbeurteilung von Hepatitis B-Infektionen nützlich, da sie bevorzugt mit Hepatitis B-Viruspartikeln und weniger mit den 20 nm Hepatitis B-Oberflächenantigenpartikeln reagieren. Prä-S(1)-halige Antigene sind nützliche Reagenzien, um humorale und celluläre Immunreaktionen gegen diese Antigene zu testen. Diese Immunreaktionen können zum Beispiel auch von großer Bedeutung für die Ausheilung einer Hepatitis B sein. Antikörper gegen Prä-S(1) Protein sind gegebenenfalls auch für die Elimination von Hepatitis B-Viren aus biologischem Material geeignet. Darüberhinaus sind Prä-S(1)-haltige Mittel gegebenenfalls auch für die Behandlung bereits bestehender Hepatitis B-Virus-Infektionen nützlich.

Peptide, die Untereinheiten der Prä-S(1)-Aminosäuresequenz enthalten, oder ausschließlich aus solchen Untereinheiten bestehen, und beispielsweise aus dem Prä-S(1)-Protein (Protein, welches die Prä-S(1)-Aminosäuresequenz enthält) gemäß Anspruch 8, worin X=OH ist oder eine andere der angegebenen Bedeutungen besitzt, durch Spaltung entstehen, sind ebenfalls in gleicher

...

Weise gegen Hepatitis B immunogen wirksam und daher Teil der Erfindung. Solche Peptide, die ausschließlich Untereinheiten der Prä-S(1)-Aminosäuresequenz darstellen, sind beispielsweise die folgenden Oligopeptide:
H-PheProAspHisGlnLeuAspProAla-OH; H-AsnAsnProAspTrpAsp PheAsnPro-OH; H-ThrAsnArgGlnSerGlyArgProThr-OH; H-AlaAsnArgGlnSerGlyArgProThr-OH; H-ProProProAlaSerThr AsnArgGlnSerGlyArgGlnProThrPro-OH oder H-ProProProAlaSer AlaAsnArgGlnSerGlyArgGlnProThrPro-OH.

Die hier in Frage kommenden Untereinheiten der Prä-S(1)-Aminosäuresequenz müssen mindestens aus 6 Aminosäuren bestehen.
Größere Peptide, die die vorstehend angegebenen Aminosäuresequenzen enthalten, sind ebenfalls immunogen gegen Hepatitis B wirksam.

Das Hepatitis B-Virus (HBV) enthält ein Oberflächenprotein mit 39 000 Dalton scheinbarem Molgewicht (P39) und ein Glykoprotein mit 42 000 Dalton (GP42). GP42 und P39 haben dieselbe Proteinsequenz, GP42 enthält aber zusätzlich eine N-glykosidisch gebundene Kohlenhydratseitenkette. P39/GP42 ist co-terminal mit dem bekannten viralen Oberflächenprotein GP33 (STIBBE & GERLICH, Virol. 123, 436-442, 1982, und J.Virol. 46, 626-628, 1983). Co-terminal heißt, daß die gesamte Aminosäuresequenz von GP33 im carboxy-terminalen Teil von P39/GP42 enthalten ist. Zusätzlich enthält P39/42 am aminoterminalen Teil eine Sequenz von 108 oder 119 Aminosäuren, die erfindungsgemäß als Prä-S(1)-Sequenz (Prä-S(1)-Aminosäuresequenz) bezeichnet wird.

Die Struktur von entsprechenden Prä-S(1)-Polypeptiden ergibt sich aus den bekannten DNA-Sequenzen verschiedener Hepatitis-Virus B-Isolate. Zwischen den Hepatitis B-Virus-Subtypen gibt es gerade in der Prä-S(1)-Sequenz zahlreiche Aminosäureaustausche. Die genetische Grundstruktur, die die Prä-S(1)-Sequenz als amino-terminalen Teilabschnitt der großen Oberflächenproteine P39/GP42 definiert, ist jedoch bei allen bekannten Isolaten von Hepatitis B-

...

Virus gleich. Die verschiedenen Oberflächenproteine werden alle von einer kontinuierlichen Sequenz von Basentripletts (= Codons) codiert, die mit einem ersten Startcodon beginnt und mit einem Stopcodon endet. Das Startcodon besteht aus dem Triplett ATG, das für den Beginn der Proteinbiosynthese nötig ist und zugleich die Aminosäure Methionin als Aminoende des Proteins codiert. Die Biosynthese des Proteins bricht mit einem der Stopcodons, TAA, TGA, TAG ab. Im Normalfall beginnt die Proteinbiosynthese mit dem ersten ATG-Codon einer codierenden Sequenz. Dies ist auch für das P39/GP42 des Hepatitis B-Virus der Fall, so daß ein Protein von 389 oder 400 Aminosäuren entsteht.

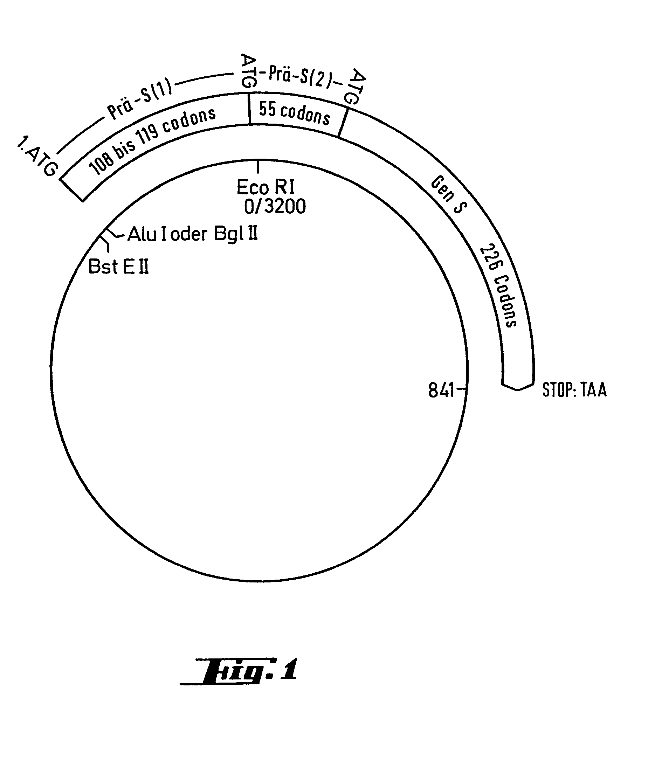

Als Besonderheit des Hepatitis B-Virus werden jedoch auch zwei spätere Startcodons als fakultative Startpunkte der Proteinbiosynthese von infizierten Zellen verwendet. Ein ATG-Triplett, das 108 bis 119 Tripletts nach dem ersten Startcodon (je nach Subtyp) liegt, dient als Aminoende des GP33/36. Die Sequenz zwischen diesem zweiten und dem ersten Startcodon wird als Prä-S(1)-Sequenz definiert. Noch weitere 55 Tripletts später befindet sich ein drittes Startcodon, das das Aminoende des Hepatitis B-Virus-Oberflächen-Hauptproteins P24/GP27 (auch als Gen S bezeichnet) festlegt. Die Sequenz von 55 Codons zwischen dem zweiten und dritten Startcodon wird als Prä-S(2)-Sequenz definiert. Die Lage der Prä-S(1)- und Prä-S(2)-Nucleotidsequenzen in der ringförmigen Hepatitis B-DNA sowie die Schnittstellen für die Prä-S(1)-Nucleotidsequenz wird in Fig. 1 gezeigt. Die EcoRI-Schnittstelle wird in üblicher Weise als Anfang, das heißt mit 0 bezeichnet.

Die Prä-S(1)-Sequenz bezeichnet eine Aminosäuresequenz von 108 oder 119 Aminosäuren sowie die entsprechende für

...

die Codierung dieser Aminosäuresequenz verantwortliche Nucleotidsequenz der Hepatitis B-Virus-DNA. Die Prä-S(1)-Nucleotidsequenz ist die Folge von 108 oder 119 Codons, die maximal 400 oder 389 Codons vor dem Stopcodon des offenen Ableserahmen beginnt, der das HBsAg codiert und 281 Codons vor dem Stopcodon desselben offenen Ableserahmens endet. Diese Prä-S(1)-Nucleotidsequenz codiert die entsprechende Prä-S(1)-Aminosäuresequenz. Die Prä-S(2)-Nucleotidsequenz besteht aus 55 Codons und schließt sich unmittelbar an die Prä-S(1)-Nucleotidsequenz an; sie beginnt mit dem Startcodon, der 281 Codons vor dem Stopcodon des offenen Ableserahmens für das HBsAg liegt (Codon ATG das Methionin codiert) und endet nach 55 weiteren Codons desselben Ableserahmens. Die entsprechende Prä-S(2)-Aminosäuresequenz ist beispielsweise die folgende: MetGlnTrpAsnSerThrAlaPheHisGlnAlaLeuGlnAsp ProArgValArgGlyLeuTyrPheProAlaGlyGlySerSerSerGlyThrValAsn ProAlaProAsnIleAlaSerHisIleSerSerIleSerAlaArgThrGlyAspPro ValThrAsn.

Unter HBsAg codierenden offenen Ableserahmen (siehe Anspruch 1) wird hierbei der Teil der Hepatitis B-Virus-DNA verstanden, welcher beispielsweise in Viral Hepatitis 1981, International Symposium, The Franklin Institute Press, Philadelphia 1982 auf Seite 82 erwähnt ist und aus den zwei Bezirken "Region Pre S" und "Gen S" besteht. Diese Region Pre S besteht aus der erfindungsgemäßen Prä-S(1)-Sequenz und der sich hieran anschließenden Prä-S(2)-Sequenz, wobei sich das Gen S (Gen für = Protein P24/GP27) unmittelbar an die Prä-S(2)-Sequenz anschließt (siehe auch Viral Hepatitis, Laboratory and Clinical Science, F. und J. Deinhardt Editors Marcel Dekker, New York, Basel 1983, Seite 71). Die Prä-S(1)-Aminosäuresequenz ist also die Folge von 108 oder 119 Codons der Hepatitis B-Virus-DNA, die mit dem ersten Startcodon des HBsAg codierenden Ableserahmens beginnt und 281 Codons vor dessen Stopcodon endet.

...

Unter Hepatitis B-Oberflächenantigen (HBsAg) im Rahmen dieser Erfindung wird folgendes verstanden: HBsAg ist die Antigeneigenschaft der 20 nm Hepatitis B-Oberflächenpartikel, die im wesentlichen aus den Proteinen P24 beziehungsweise GP27 bestehen. Diese Proteine P24 beziehungsweise GP27 sind zum Beispiel in Peterson et al, Proc.Nat. Acad.Sci., USA, Band 74 (1977), Seiten 1530-34 beschrieben. Das Protein P24 wird beispielsweise von den Nucleotiden 161 bis 841 gemäß Fig. 4 auf Seite 71 des oben zitierten Buches Viral Hepatitis, Laboratory and Clinical Science beschrieben. Der offene Ableserahmen, der dieses HBsAg codiert, beginnt gemäß der zuvor erwähnten Fig. 1, jedoch viel früher und zwar mit dem Nucleotid 2859.

Die Prä-S(1)-Aminosäuresequenz beziehungsweise Peptide, die diese Prä-S(1)-Aminosäuresequenz enthalten, sind auch dadurch charakterisiert, daß sie spezifisch mit dem monoklonalen Antikörper MA18/7 reagieren und diesen binden.

Erfindungsgemäße Polypeptide, die im wesentlichen aus der Prä-S(1)-Aminosäuresequenz bestehen, können außer dieser Prä-S(1)-Sequenz noch bis zu 12 weitere natürliche Aminosäuren enthalten, wobei sich diese zusätzlichen 1 - 12 Aminosäuren vorzugsweise am Carboxy-terminalen Ende der Prä-S(1)-Aminosäuresequenz befinden. Insbesondere kommen hier die Aminosäuren beziehungsweise die Aminosäuresequenzen in Frage, die in Anspruch 8 für X angegeben sind. Größere Polypeptide, welche die erfindungsgemäße Prä-S(1)-Aminosäuresequenz enthalten, können beispielsweise an ihrem Carboxy-terminalen Ende noch 12 - 1000 und an ihrem Amino-terminalen Ende noch 1 - 1000, vorzugsweise 1 - 500, insbesondere 1 - 250 weitere natürliche Aminosäuren enthalten. Insbesondere können solche größeren Polypeptide an ihrem Carboxy-terminalen Ende die ganze Prä-S(2)-Aminosäuresequenz enthalten oder auch diese Prä-S(2)-Aminosäure-

...

- 9 -

sequenz plus Aminosäuren beziehungsweise Peptidteile, die von dem sich an den Prä-S(2)-Teil anschließenden Gen S stammen.

Die DNA-Fragmente für die Synthese des Prä-S(1)-Polypeptids werden durch Schnitte der Hepatitis B-Virus-DNA mit beispielsweise folgenden Restriktionsenzymen erzeugt:
Mit BstEII und nachfolgender Behandlung mit Exonucleasen, zum Beispiel Bal31 (alle Subtypen), AluI (ad Subtypen) oder BglII (ay Subtypen) oder deren Isoschizomeren als Schnitt vor der Prä-S(1)-Sequenz; die Prä-S(1)-Sequenz beginnt insbesondere dort, wo nach diesem Schnitt erstmalig die Aminosäure Methionin kommt, das heißt sie wird beispielsweise in der Hepatitis B-DNA durch das erste Methion-Codon (ATG) nach der Schnittstelle mit den Enzymen BstEII + Bal31 markiert. Zusätzliche ATG-Tripletts innerhalb der Prä-S(1)-Sequenz sind von Subtyp zu Subtyp nicht konserviert und haben keine Funktion als Startcodon, sondern codieren lediglich Methionin innerhalb der Prä-S(1)-Sequenz. Der zweite Schnitt erfolgt beispielsweise mit EcoRI als Schnitt nach der Prä-S(1)-Sequenz; das Ende der Prä-S(1)-Sequenz ist also beispielsweise dort, wo vor diesem EcoRI-Schnitt das erste Codon für die Aminosäure Alanin steht. Als Schnitt nach der Prä-S(1)-Sequenz und vor Gen S sind darüberhinaus noch zahlreiche weitere Enzyme je nach Subtyp geeignet, wie zum Beispiel XhoI (bei Subtyp ay), BamHI (bei Subtyp $adw_2$).

Bei den Polypeptiden mit der Aminosäuresequenz Prä-S(1)-X hat X insbesondere folgende Bedeutungen:

1. Met-Gln-Trp oder Met-His-Trp als Rest der Prä-S(2)-Sequenz bis zur EcoRI-Schnittstelle.

...

2. Met-Gln-Trp-Y oder Met-His-Trp-Y, wobei Y ein für die Erfindung nicht wesentlicher Anteil von 1 bis 50, vorzugsweise 1 bis 30, insbesondere 1 bis 12 natürlichen Aminosäuren ist, die durch den Vector zufällig codiert werden bis ein Stopcodon in der DNA-Sequenz auftritt; beispielsweise ist Y LeuProArgAlaPheArg-OH.

3. Wird die Prä-S(1)-Sequenz beispielsweise in die PvuII-Stelle des Plamids pGL101 (beziehungsweise pBR322) eingesetzt, kann X außerdem zum Beispiel folgende Bedeutungen haben: CysLeuAlaArgPheGlyAspAspGlyGlu AsnLeu-OH, (12 Aminosäuren) AlaSerArgValSerValMet ThrValLysThrSerAspThrCysSerSerArgArgArgSerLeuLeu ValCysLysArgMetProGlyAlaArgLysProValArgAlaArgGln ArgValLeuAlaGlyValGlyAlaGlnPro-OH.

4. Statt mit EcoRI kann die HBV-DNA auch mit einem Enzym geschnitten werden, dessen Erkennungssequenz näher am Stopcodon des offenen Ableserahmens für HBsAg liegt. Das erhaltene Polypeptid enthält als Sequenz X dann größere Teile der Prä-S(2)-Sequenz oder die gesamte Prä-S(2) und noch aminoterminale Teile des Gen S. Als Beispiel kann für den Subtyp adw$_2$ ein Schnitt mit BamHI genannt werden, für die Subtypen mit ay XhoI, wobei das exprimierte Protein dann außer der Prä-S(1)-Sequenz auch noch größere aminoterminale Anteile der Prä-S(2)-Sequenz enthält. Ein Schnitt mit beispielsweise XbaI würde zu einem Protein führen, das die gesamte Prä-S(2)-Sequenz und aminoterminale Bereiche des Gen S enthält.

Weiterhin sind ganz allgemein auch Proteine mit höherem Molekulargewicht als beispielsweise die Polypeptide, die im wesentlichen aus der Prä-S(1)-Aminosäuresequenz

...

bestehen, immunogen gegen Hepatitis B wirksam, sofern sie nur an irgendeiner Stelle die Prä-S(1)-Sequenz oder Fragmente dieser Sequenz eingebaut enthalten.

Die Herstellung von derartigen höher molekularen Proteinen mit der Prä-S(1)-Sequenz oder Fragmenten der Prä-S(1)-Sequenz kann zum Beispiel nach folgender Methode erfolgen:
Die Prä-S(1)-Nucleotidsequenz kann so mit einem beliebigen anderen Gen durch DNA-Ligation in Phase zusammengefügt werden, daß die Proteinsynthese kontinuierlich von der Prä-S(1)-Sequenz auf das andere Gen übergeht. Das zweite Gen kann ein beispielsweise bekanntes bakterielles Enzym codieren, zum Beispiel beta-Galaktosidase oder ein Protein, das selbst als Impfstoff interessant ist, zum Beispiel Diphterie- oder Tetanustoxin. Das Fusionsprodukt ist wegen seiner Größe im allgemeinen immunogener und wegen seiner teilweise bakteriellen Herkunft in der Bakterienzelle eventuell stabiler.

Es ist auch möglich, die Prä-S(1)-Sequenz innerhalb eines bakteriellen Strukturgens einzusetzen. Es sind Vector-Plasmide vom pUR- oder pUC-Typ verfügbar, die Restriktionsschnittstellen innerhalb einer codierenden Sequenz für ein Fragment der beta-Galactosidase enthalten (Gene 19 (1982), 259).

In diese oder ähnliche Plasmide können jegliche Fragmente der HBV-DNA, speziell auch solche mit wesentlichen Teilen der Prä-S(1)-Sequenz eingesetzt werden. Wenn durch geeignete Behandlung der HBV DNA-Fragmente, beispielsweise Verkürzung mit Bal31 oder Verlängerung um einige Basen mit dem Enzym terminale Nucleotidtransferase, in einem Teil der rekombinierten DNA ein phasenrichtiger Einbau erzielt wird, können Klone mit einer solchen DNA, ein Fusionsprotein mit Prä-S(1)-Sequenzanteilen exprimieren.

Hierbei ist es nicht nötig, daß das HBV-DNA-Fragment

...

- 12 -

noch das Startcodon der Prä-S(1)-Sequenz enthält.
Die eingesetzten HBV-DNA-Fragmente können sehr große
Bereiche umfassen, zum Beispiel auch noch Anteile der
Prä-S(2)-Region und des Gens S oder auch nur kleine
Ausschnitte der Prä-S(1)-Region. Sehr kleine Ausschnitte
mit zum Beispiel nur 27 Nucleotiden können für diesen
Zweck bevorzugt durch chemische Nucleotidsynthese hergestellt werden. Zum Zweck der größeren Stabilität
und leichteren Isolierbarkeit werden auch solche kleinen
Genfragmente mit größeren Genen fusioniert.

...

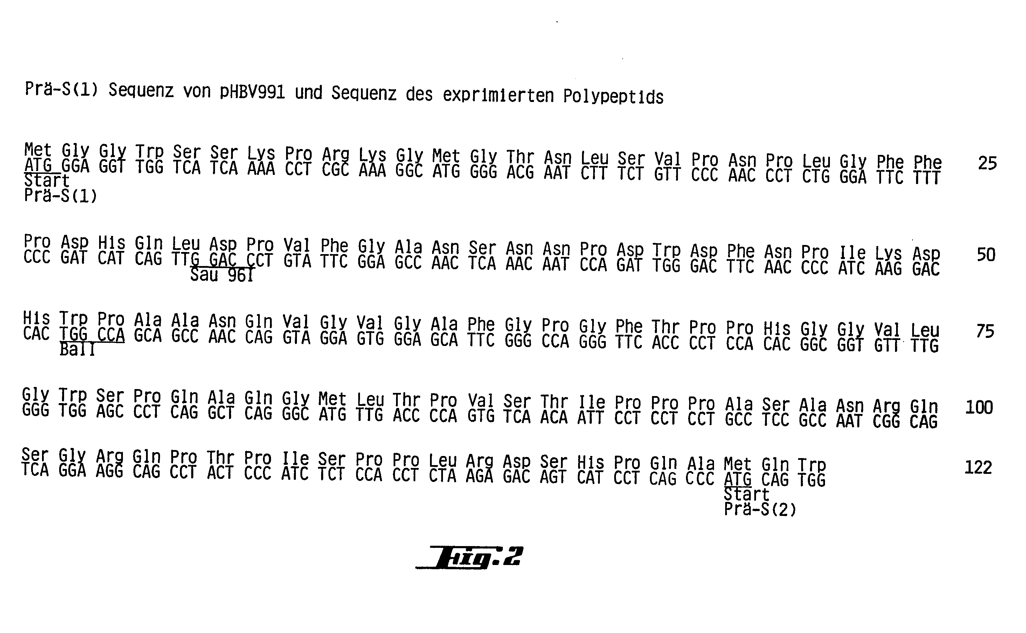

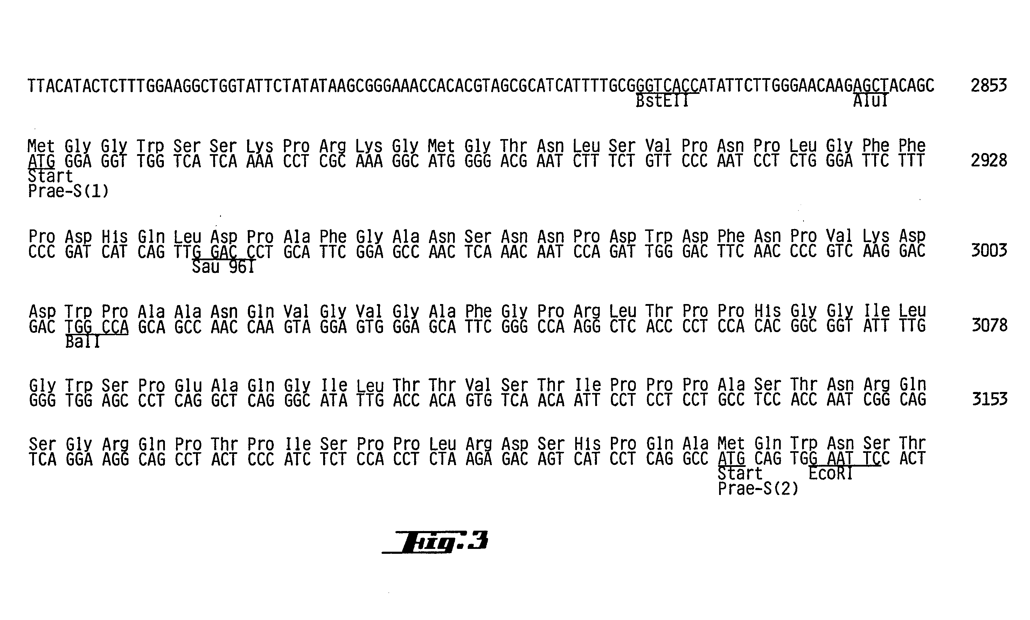

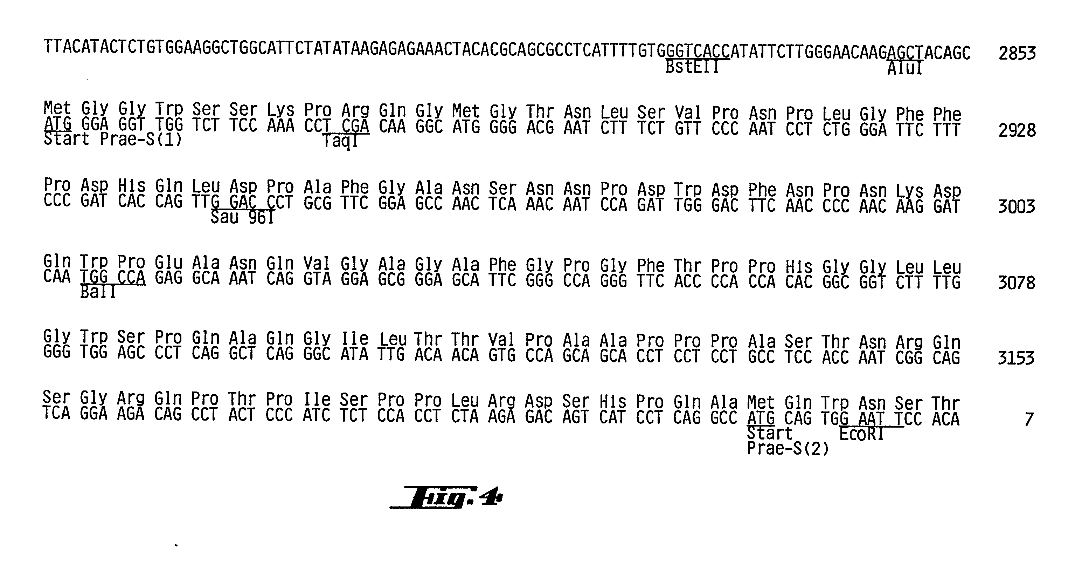

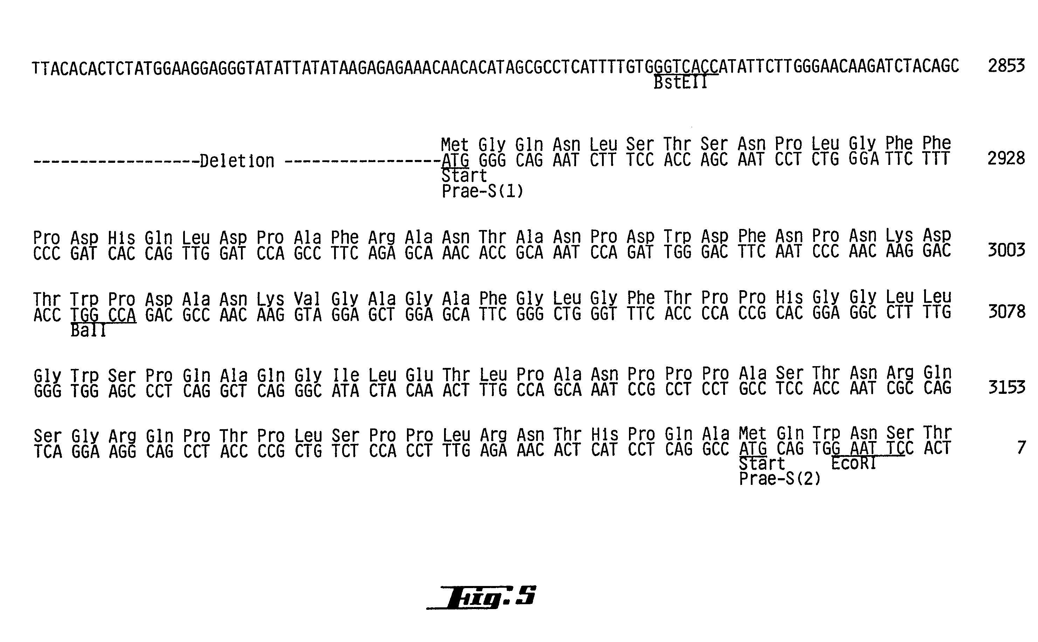

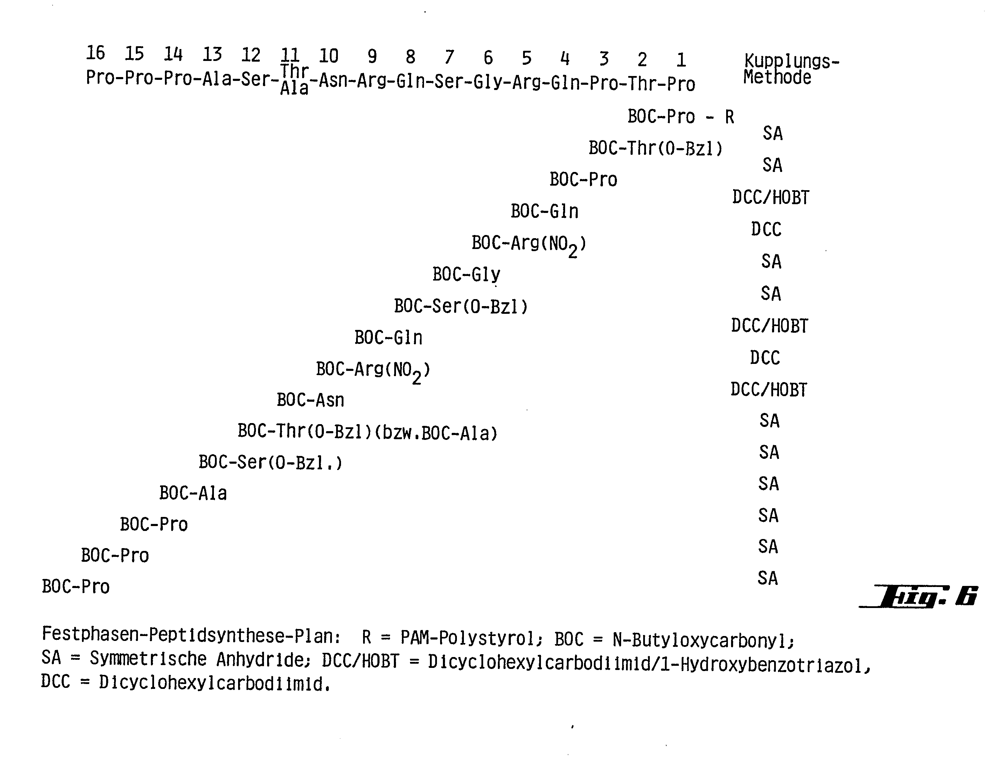

In den Fig. 2, 3, 4 und 5 sind beispielhaft Prä-S(1)-Nucleotidsequenzen von verschiedenen Hepatitis B-Virus-DNA Isolaten verschiedenen Subtyps (zum Beispiel Subtyp ayw, adw$_2$, adr) einschließlich wesentlicher flankierender Bereiche sowie die diesen Prä-S(1)-Nucleotidsequenzen entsprechenden Prä-S(1)-Aminosäuresequenzen dargestellt. Fig. 2 und Fig. 3 betreffen zwei verschiedene Isolate des Subtyps adw$_2$ der Hepatitis B-DNA, Fig. 4 den Subtyp adr und Fig. 5 den Subtyp ayw. Hierbei wurde die EcoRI Restriktionsschnittstelle als Nullpunkt der DNA-Sequenz angenommen. Die Prä-S(1)-Aminosäuresequenz von Fig. 2 entspricht dem Peptid der Formel I (X=OH). Die Prä-S(1)-Aminosäuresequenz von Fig. 3 entspricht dem Peptid der Formel II, die Prä-S(1)-Aminosäuresequenz von Fig. 4 entspricht dem Peptid der Formel III und die Prä-S(1)-Aminosäuresequenz von Fig. 5 entspricht dem Peptid der Formel IV (X jeweils = OH).

Die Erfindung betrifft daher auch die Nucleinsäurefragmente, die aus der DNA des Hepatitis B-Virus herausgeschnitten werden können, welche den Teil des Gens enthalten, der in der Lage ist, für die ersten 119 beziehungsweise 108 Aminosäuren des Proteins mit Mol-Gewicht 39 000 Dalton zu kodieren. Dieses Protein wird zum Beispiel gemäß Beispiel II erhalten.

In dieser Hinsicht betrifft die Erfindung demnach ein Nucleinsäure-Fragment mit etwa 324 bis 357 Nucleotiden, das insbesondere dadurch gekennzeichnet ist, daß es in der Lage ist, für eine immunogene Peptidsequenz zu kodieren, welche ihrerseits in der Lage ist, in vivo die Produktion von Antikörpern zu induzieren, die gegen das Hepatitis B-Virus aktiv sind, wobei diese Peptidsequenz im wesentlichen die in den Figuren 2, 3, 4 und 5 dargestellte Struktur aufweist, oder für irgendeine Peptidsequenz mit äquivalenten immunogenen Eigenschaften.

...

Die Erfindung betrifft ebenfalls einen Vektor im Hinblick auf die Expression der genannten Nucleotidsequenz in einem Mikroorganismus oder in eukaryotischen Zellen mit der Maßgabe, daß bei der genetischen Verknüpfung die Ablesephase des Prä-S(1)-Gens beibehalten wurde.

Die erfindungsgemäß verwendeten Nucleotidsequenzen weisen im Verhältnis zueinander eine Veränderlichkeit auf, die bei ihrer Expression zur Entstehung von Determinanten führt, die je nach dem Subtyp des Hepatitis B-Virus variieren (Subtypen $adw_2$, adr, ayw und sonstige Varianten).

Bei der Peptidsequenz gemäß den Figuren 2, 3, 4 und 5 ist die erste Aminosäure der vorstehend erläuterten Sequenz: Methionin, N-terminal und die Aminosäure des entgegengesetzten Endes Alanin, C-terminal.

Die Erfindung betrifft daher insbesondere auch die in den Figuren 2, 3, 4 und 5 wiedergegebene Nucleotidsequenz, die für eine solche Peptidsequenz kodiert, wie sie zum Beispiel in Anspruch 6 wiedergegeben ist, oder für irgendeine analoge Peptidsequenz, die mit äquivalenten immunogenen Eigenschaften versehen ist.

Es versteht sich von selbst, daß mit dem vorstehend erwähnten Begriff "äquivalente Peptidsequenz" jede Peptidsequenz gemeint ist, in der bestimmte Teile nicht streng identisch mit den entsprechenden Teilen der in den Figuren 2, 3, 4 und 5 wiedergegebenen Peptidsequenz sein können, wobei diese Variationen auf lokale Mutationen zurückzuführen sein können, welche den allgemeinen immunogenen Charakter des Proteins nicht beeinflussen, oder auf Modifikationen der Struktur, die zu den unterschiedlichen Serotypen führen, unter denen die Proteine der in Frage stehenden Art in Erscheinung zu treten vermögen.

...

0180012

Insbesondere betrifft die Erfindung auch folgende Peptidsequenzen, die Untereinheiten der Prä-S(1)-Aminosäuresequenz darstellen:

Peptid A:     PheProAspHisGlnLeuAspProAla
Peptid B:     AsnAsnProAspTrpAspPheAsnPro
Peptid Ca:    ThrAsnArgGlnSerGlyArgProThr
Peptid Cb:    AlaAsnArgGlnSerGlyArgProThr
Peptid Cc:    ProProProAlaSerThrAsnArgGlnSerGlyArgGlnProThrPro
Peptid Cd:    ProProProAlaSerAlaAsnArgGlnSerGlyArgGlnProThrPro

In den vorstehend aufgeführten Peptiden (dies gilt auch für alle übrigen in dieser Anmeldung genannten Peptide beziehungsweise Peptidfragmente) ist jeweils die erste linksstehende Aminosäure Amino-terminal (zum Beispiel bei Peptid A Phe) und die letzte rechtsstehende Aminosäure Carboxy-terminal (zum Beispiel bei Peptid A Ala).

Die Herstellung von kleineren Peptidfragmenten aus der Prä-S(1)-Aminosäuresequenz kann nach den üblichen Verfahren zur Herstellung synthetischer Peptide erfolgen. Beispielsweise kann man das Peptid Cc beziehungsweise Cd ausgehend von der Carboxy-terminalen Aminosäure nach dem Verfahren c) des Verfahrensanspruchs herstellen.

...

- 16 -

Die Erfindung betrifft auch die Produkte, die sich aus der Anheftung der erfindungsgemäßen Polypeptide (zum Beispiel gemäß Anspruch 8) sowie von den vorstehend angegebenen Untereinheiten solcher Polypeptide (zum Beispiel Oligopeptide des Anspruchs 7 beziehungsweise Fragmente von Polypeptiden gemäß Anspruch 8) an ein größeres Trägermolekül, insbesondere vom Polypeptid- oder Proteintyp, ergeben, ferner Mittel, welche solche Peptide in Form von Verknüpfungsprodukten enthalten, insbesondere zusammen mit einem pharmazeutisch verträglichen Träger, sowie insbesondere Impfstoffe gegen die Hepatitis B. Die pharmazeutischen Träger sind in an sich üblicher Weise der ausgewählten Verabreichungsart angepasst, die insbesondere oral, parenteral, rektal oder durch Aufsprühen auf die Schleimhäute, insbesondere der Nase, erfolgen kann.

Die Polypeptide gemäß Anspruch 7 oder 8 sowie Fragmente dieser Polypeptide können zum Beispiel auch nach den üblichen Verfahren der Peptidsynthese hergestellt werden.

Die Erfindung betrifft ferner noch die DNA-Sequenzen, die in der Lage sind, beispielsweise für die Herstellung von Fragmenten der Polypeptide gemäß Anspruch 8 zu kodieren.

Es handelt sich:

- Für das Peptid I insbesondere um die Polynucleotidsequenz der Formel TTT CCC GAT CAT CAG TTG GAC CCT;

- Für das Peptid II insbesondere um die Polynucleotidsequenz der Formel AAC AAT CCA GAT TGG GAC TTC AAC CCC;

- Für die Peptide IIIc und IIId um die Polynucleotidsequenz der Formel CCT CCT CCT GCC TCC G*CC AAT CGG CAG TCA GGA AGG CAG CCT ACT CCC oder um Ausschnitte von mindestens 6 Tripletts

...

* oder A

oder in jedem dieser Fälle um das zu den drei jeweiligen vorstehenden Polynucleotiden komplementäre Polynucleotid, oder auch um irgendein Polynucleotid in dem jedes der Tripletts durch irgendein analoges Triplett ersetzt sein kann, das in der Lage ist, für den Einbau der gleichen Aminosäure zu kodieren:

Die Nucleinsäure der Erfindung kann ferner auch nur einen der zwei wechselseitig komplementären Stränge der entsprechenden DNA-Sequenz gemäß Fig. 1 enthalten.

Die Erfindung betrifft natürlich auch die äquivalenten, einsträngigen oder doppelsträngigen Prä-S(1)-Nucleotidsequenzen, darunter insbesondere den codierenden Strang, die entsprechende doppelsträngige DNA oder die entsprechenden Boten-RNAs, insbesondere diejenige, die durch die entsprechenden Komplementärketten der Nucleotide wiedergegeben wird.

Ebenfalls gehören zur Erfindung die Prä-S(1)-Nucleotid-ketten, die sich von den vorstehenden durch bestimmte Tripletts oder kurze Triplettsequenzen unterscheiden, mit der Maßgabe, daß diese Nucleotidsequenzen befähigt bleiben, für ein Polypeptid zu kodieren, das die immunogenen Eigenschaften der Prä-S(1)-Polypeptide zum Beispiel gemäß Anspruch 8 bewahrt. Allgemein handelt es sich um Prä-S(1)-Nucleotidketten, die gegebenenfalls nach Denaturierung der doppelsträngigen DNA zur Erzeugung der entsprechenden einsträngigen Nucleinsäuren die Fähigkeit behalten, sich auf mindestens etwa 90 % ihrer Länge mit einem entsprechenden komplementären DNA-Strang zu hybridisieren.

Die erfindungsgemäße Prä-S(1)-DNA ist für den Einbau in einen Vektor bestimmt, der ihre Expression in einem

...

Bakterium und in eukaryotischen Zellen (zum Beispiel Hefezellen) ermöglicht, insbesondere im Hinblick auf die Herstellung eines Proteins oder eines Peptids, das in der Lage ist, in einem lebenden Wirtsorganismus die Herstellung von Antikörpern zu induzieren, die gegen das Virus der Virushepatitis B aktiv sind. Das aus der Übersetzung der Nucleotidsequenz gemäß der Erfindung hervorgehende Protein oder Peptid kann als Impfstoff oder als Hilfsmittel zur Diagnose verwendet werden.

Weitere Kennzeichen der Erfindung werden sich noch aus der Beschreibung ergeben, die sich auf die Herstellung, die Identifizierung und Gewinnung der Prä-S(1)-DNA-Fragmente sowie der Prä-S(1)-Peptide gemäß der Erfindung bezieht. Natürlich wird auf die Zeichnungen Bezug genommen, deren Figuren bereits in den vorangehenden Ausführungen in Betracht gezogen wurden.

Die Erfindung betrifft auch besondere Vektoren, die die Expression der vorstehend beschriebenen Prä-S(1)-Nucleotidsequenzen ermöglichen, insbesondere in Form eines Hybridproteins, in welchem ein Proteinfragment, das die immunologischen Eigenschaften der Polypeptide gemäß den Ansprüchen oder von Fragmenten hiervon aufweist, an ein Trägermolekül gebunden ist, wobei dem Ganzen immunogene oder immunoreaktive Eigenschaften verliehen werden, die es dazu befähigen, die Produktion von Antikörpern zu induzieren, die im Wirtsorganismus, in den dieses Protein vorher eingebracht wurde, eine Schutzwirkung gegen die virale Infektion ausüben.

Insbesondere betrifft die Erfindung den Vector Plasmid pDR540 (Hersteller Pharmacia PL Biochemicals). Dieser Vector enthält als wesentliches Strukturmerkmal den "tac"-Promoter (Gene 20 (1982), 231), der eine hoch-

...

wirksame Signalsequenz für die Auslösung der Proteinsynthese darstellt. Hinter dem tac-Promoter enthält
pDR540 eine BamHI Schnittstelle. Für die Zwecke der
Erfindung wurden die Einzelstrangenden der Schnittstelle
mit einer Einzelstrang spezifischen Nuclease entfernt.
Mischt man das so modifizierte Plasmid mit einem DNA-
Fragment, beispielsweise dem Prä-S(1)-Fragment, und
setzt das Enzym DNA-Ligase zu, entsteht rekombinierte
DNA, die immer dann zur Produktion von Proteinen führen
kann, wenn ein Startcodon aus dem eingesetzten DNA-
Fragment in der Nähe des tac-Promoters liegt.

...

- 20 -

Zu dem Verfahren a):

Als Restriktionsnuclease, die die Nucleotidsequenz GAATTC spaltet, kommt in Frage EcoRI.

Die Behandlung mit diesem Enzym erfolgt in wässrigem Medium bei Temperaturen zwischen 34 und 40° C, vorzugsweise 36° und 38° C bei pH-Werten zwischen 7,0 und 8,0, vorzugsweise 7,4 und 7,6; Zeit: 15 Minuten bis 16 Stunden, vorzugsweise 1 bis 2 Stunden.
Die Einstellung des erforderlichen pH-Wertes erfolgt beispielsweise durch Puffer, wie: 100 mM Tris-HCl; NaCl 50 mM; $MgCl_2$ 10 mM.

Beendigung der Enzymreaktionen beispielsweise durch Zusatz von: 250 mM Natrium-EDTA auf eine Endkonzentration von 25 mM.

Die Inaktivierung dieses Enzyms erfolgt zum Beispiel durch Erhitzen auf 60 bis 80, vorzugsweise 64 bis 66° C, während 5 bis 30 Minuten, vorzugsweise 10 bis 15 Minuten.

Als Restriktionsnucleasen, die die Nucleotidsequenz AGCT spalten, kommen zum Beispiel in Frage: AluI oder Isoschizomere hiervon, wie OxaI.

Die Behandlung mit AluI erfolgt in wässrigem Medium bei Temperaturen zwischen 35 und 40° C, vorzugsweise 36 und 38° C bei pH-Werten zwischen 7,0 und 8,0, vorzugsweise 7,5 und 7,7; Zeit: 15 Minuten bis 16 Stunden, vorzugsweise 1 bis 2 Stunden.

Die Einstellung des erforderlichen pH-Wertes erfolgt beispielsweise durch Puffer, wie: 6 mM Tris-HCl; 50 mM NaCl; 6 mM $MgCl_2$; 10 mM Dithiothreitol.

...

Beendigung der Enzymreaktionen beispielsweise durch Zusatz von: 250 mM NaEDTA auf 10 mM Endkonzentration.

Die Inaktivierung dieses Enzyms erfolgt zum Beispiel durch Erhitzen auf 60 bis 80, vorzugsweise 64 bis 66° C, während 5 bis 30, vorzugsweise 5 bis 10 Minuten.

Als Restriktionsendonucleasen, die die Nucleotidsequenz AGATCT spalten, kommt zum Beispiel in Frage: BGl II.

Die Behandlung mit diesem Enzym erfolgt in wässrigem Medium bei Temperaturen zwischen 34 und 40° C, vorzugsweise 36 und 38° C bei pH-Werten zwischen 9,0 und 10,0, vorzugsweise 9,4 und 9,6; Zeit: 15 Minuten bis 16 Stunden, vorzugsweise 1 bis 2 Stunden.

Die Einstellung des erforderlichen pH-Wertes erfolgt beispielsweise durch Puffer, wie: 20 mM Glycin-NaOH; 10 mM $MgCl_2$; 7 mM beta-Mercaptoethanol.

Beendigung der Enzymreaktionen beispielsweise durch Zusatz von: 250 mM NaEDTA auf 20 mM Endkonzentration.

Die Inaktivierung dieses Enzyms erfolgt zum Beispiel durch Erhitzen auf 60 bis 80, vorzugsweise 64 bis 66° C, während 5 bis 30, vorzugsweise 5 bis 10 Minuten.

Als Restriktionsnucleasen, die die Nucleotidsequenz GGTNACC spalten (N kann ein beliebiges Nucleotid A, G, T oder C sein), kommen zum Beispiel in Frage: BstEII oder Isoschizomere dieses Enzyms: AspAI, BstPI, EcaI.

Die Behandlung mit BstEII erfolgt in wässrigem Medium bei Temperaturen zwischen 55 und 65, vorzugsweise 58 und 60° C bei pH-Werten zwischen 7,0 und 8,0, vor-

...

zugsweise 7,5 und 7,7; Zeit: 15 Minuten bis 16 Stunden, vorzugsweise 1 bis 2 Stunden.

Die Einstellung des erforderlichen pH-Wertes erfolgt beispielsweise durch Puffer, wie: 100 mM Tris-HCl; 50 mM KCl; 5 mM $MgCl_2$.

Beendigung der Enzymreaktionen beispielsweise durch Zusatz von: 250 mM NaEDTA auf 10 mM Endkonzentration.

Das Restriktionsenzym EcoRI und dessen Isoschizomere spalten hinter dem Start der Prä-S(2)-Sequenz, das heißt hinter dem Start des Nucleotidteils der Hepatitis B-DNA der die bekannten Oberflächenproteine mit den Mol-Gewichten 33 000 beziehungsweise 36 000 Dalton kodiert. Die anderen vorstehend erwähnten drei Typen von Restriktionsenzymen (BstEII, AluI, BGl II und deren Isoschizomere) spalten vor dem Startpunkt der Prä-S(1)-Sequenz.

Die Behandlung der Hepatitis B-DNA mit den beiden Enzymtypen EcoRI einerseits und BstEII, AluI oder BGl II andererseits kann gleichzeitig erfolgen oder nacheinander, wobei die Reihenfolge beliebig ist. Beispielsweise kann man zuerst mit BstEII oder einem Isoschizomeren hiervon oder zuerst mit AluI beziehungsweise BGl II behandeln und anschließend mit dem Enzymtyp EcoRI, oder umgekehrt.

Falls bei der Spaltung mit den genannten Restriktionsenzymen DNA-Fragmente entstehen mit überstehenden 5'-Einzelstrangenden (5'-sticky-ends), wie dies zum Beispiel insbesondere bei Verwendung EcoRI und von BstEII und den entsprechenden Isoschizomeren der Fall ist, empfiehlt es sich, diese heraushängenden Enden mit einzelstrangspezifischen Nucleasen zu entfernen, das heißt in stumpfe Enden (blunt-ends) umzuwandeln.

...

Als einzelstrangspezifische Nucleasen für diese Umwandlung kommen zum Beispiel in Frage: Mung-Bean-Nuclease, Nuclease P1, Nuclease S1, Nuclease aus Neurospora crassa.

Die Behandlung mit Mung-Bean-Nuclease erfolgt zum Beispiel in wässrigem Medium bei Temperaturen zwischen 20 und 45, vorzugsweise 36 und 38° C bei pH-Werten zwischen 4 und 5, vorzugsweise 4,4 und 4,5; Zeit: 5 bis 30 Minuten, vorzugsweise 5 bis 15 Minuten.

Die Einstellung des erforderlichen pH-Wertes erfolgt beispielsweise durch Puffer, wie 60 mM Natriumacetat-Essigsäure; 100 mM NaCl; 2 mM ZnCl; 10 % Glycerin.

Beendigung der Enzymreaktion beispielsweise durch Zusatz von: 250 mM NaEDTA auf 10 mM Endkonzentration.

Die Entfernung dieser Enzyme erfolgt zum Beispiel durch Ausschütteln mit Phenol und Chloroform wie beschrieben in Molecular Cloning, A Laboratory Manual, Seite 458, Verfasser T. Maniatis, E.F. Fritsch, J. Sambrook, Cold Spring Harbor Laboratories, Cold Spring Harbor, N.Y., USA, 1982).

Die Entfernung von überstehenden Einzelstrangenden kann auch durch Behandlung mit einer doppelstrangspezifischen Exonuclease, wie Bal31, erfolgen, wobei nun nicht nur der überstehende Einzelstrang, sondern das ganze Fragment an dem Ende, wo das Startcodon für die Prä-S(1)-Sequenz liegt, weiter verkürzt wird, wodurch eine günstigere Distanz zwischen dem Startcodon der Prä-S(1)-Sequenz und der Shine-Delgarno-Sequenz erreicht wird, die möglichst 3 bis 12 Basenpaare betragen soll.

...

- 24 -

Die Behandlung mit diesem Enzym erfolgt in wässrigem Medium bei Temperaturen zwischen 20 und 40° C, vorzugsweise 25 und 35° C bei pH-Werten zwischen 7,0 und 8,5, vorzugsweise 7,5 und 8,1; Zeit: 1 bis 30 Minuten, vorzugsweise 10 bis 20 Minuten.

Die Einstellung des erforderlichen pH-Wertes erfolgt beispielsweise durch Puffer, wie: 20 mM Tris-HCl; 12 mM $MgCl_2$; 12 mM $CaCl_2$; 60 mM NaCl; 1 mM EDTA.

Beendigung der Enzymreaktion beispielsweise durch Zusatz von 250 mM NaEDTA auf 35 mM.

Die Inaktivierung dieser Enzyme erfolgt zum Beispiel durch Erhitzen auf 60 bis 75° C, vorzugsweise 60 bis 70° C während 5 bis 30 Minuten, vorzugsweise 5 bis 10 Minuten.

Weiterhin ist es gegebenenfalls erforderlich, Einzelstranglücken in den erhaltenen DNA-Fragmenten zu verschließen. Hierzu wird zusätzlich und zweckmäßig nach Behandlung mit der Exonuclease mit entsprechenden Polymerasen behandelt. Als solche Polymerase kommt zum Beispiel in Frage: Das große Fragment der DNA-Polymerase I aus E.coli nach Klenow oder reverse Transcriptase.

Die Behandlung mit dem DNA-Polymerase I-Fragment erfolgt in wässrigem Medium bei Temperaturen zwischen 6° C und 25° C, vorzugsweise 10 und 14° C bei pH-Werten zwischen 7,0 und 8.0, vorzugsweise 7,3 und 7,5; Zeit: 10 Minuten bis 16 Stunden, vorzugsweise 4 bis 16 Stunden je nach Temperatur.

Die Einstellung des erforderlichen pH-Wertes erfolgt

...

0180012

beispielsweise durch Puffer wie: 6,6 mM Tris-HCl; 6,6 mM $MgCl_2$; 5 mM NaCl; 1 mM Dithiothreitol.

Ein geeignetes Fragment wird beispielsweise erhalten durch die Behandlung der HBV-DNA mit der Restriktionsnuclease vom Typ BstEII, gefolgt von einer anschließenden Behandlung mit einer Exonuclease und von einem Schnitt mit EcoRI. Um das durch BstEII hervorgerufene Fragmentende weiter in Richtung der Prä-S(1)-Sequenz zu verschieben, empfiehlt es sich, als Alternative zum Bal31 Verdau die etwa 400 Basenpaar langen DNA-Fragmente mit der Prä-S(1)-Sequenz oder eine rekombinierte DNA aus einem solchen etwa 400 Basenpaar langen Fragment und der DNA eines Plasmids (zum Beispiel Plasmid pGL101) nochmals mit dem Restriktionsenzym AluI zu behandeln, wodurch nun ein DNA-Fragment mit der Prä-S(1)-Sequenz erhalten wird, das eine optimale Distanz von 3 bis 12 Basenpaaren zwischen dem Startcodon der Prä-S(1)-Sequenz und der Shine-Delgarno-Sequenz besitzt. Gegebenenfalls kann hierbei die rekombinierte Plasmid DNA durch Transformierung auf eine Wirtszelle (E.coli-Bakterien) mit anschließender Selektionierung und Klonierung nochmals vermehrt werden, bevor aus ihr nach Lysierung der positiven Klone das DNA-Fragment mit der Prä-S(1)-Sequenz beispielsweise durch Behandeln mit dem Enzym AluI wieder herausgeschnitten wird.

Die Isolierung der DNA-Fragmente mit der Prä-S(1)-Sequenz kann zum Beispiel in üblicher Weise erfolgen durch: Gelelektrophorese in Polyacrylamidgel von 3,5 bis 8,0 %, vorzugsweise 4 bis 6 % oder in Agarosegelen von 0,9 bis 2,0 %, vorzugsweise 1,2 bis 1,5 % mit den Puffern und Bedingungen, wie sie in "Molecular Cloning, A Laboratory Manual" (Cold Spring Harbor Laboratory, 1982), Seiten 150 bis 178 angegeben sind. Die Fragmente

...

werden bevorzugt durch Färbung mit Ethidiumbromid und Beleuchtung mit langwelligem UV-Licht sichtbar gemacht und durch Vergleich mit Fragmentserien bekannter Größe wird das gewünschte Fragment mit etwa 370 bis 390 Basen identifiziert und die Gelfläche, die dieses Fragment enthält, ausgeschnitten. Die DNA wird aus Agarose durch Electroelution in einem Dialyseschlauch herausgelöst und durch Ionenaustausch mit Diethylaminoethyl-Cellulose gereinigt. Die genauen Verfahren sind im Laboratory Manual (ibid.) beschrieben. Aus Polyacrylamidgel wird das gewünschte DNA-Fragment durch Diffusion in Puffer wie beschrieben eluiert.

...

Zur Gewinnung von Polypeptiden, die von der Prä-S(1)-Sequenz kodiert werden, werden die entsprechenden DNA-Fragmente mit der Prä-S(1)-Sequenz, die wie vorstehend beschrieben erhalten wurden, mit der DNA eines Vektors (zum Beispiel Plasmid oder virale DNA) kombiniert. Als Plasmide kommen hierfür zum Beispiel in Frage: Plasmide mit einem selektionierbaren Marker-Gen, beispielsweise dem Gen für Ampicillinresistenz und einem starken Promoter, insbesondere die Plasmide pGL101 und pDR540. Das Plasmid pGL101 ist in Form des Bakterienstammes E.coli JM101pGL101 unter der Hinterlegungsnummer DSM 3081 bei der Deutschen Sammlung von Mikroorganismen, Griesebachstraße 8, D-3400 Göttingen, hinterlegt. Plasmid pGL101 (J.Virol. 37 (1981) 683-697) enthält als Signalsequenz den lac uv 5 Promoter (Gene 20 (1982) 231) der im Normalzustand des E.coli Bakteriums durch ein Regelprotein, den lac-Repressor blockiert ist. Dieser lac-Repressor wird beispielsweise durch Isopropylthiogalactosid (IPGT), dem sogenannten Induktor, unwirksam gemacht. Nach Zusatz von IGPT wird der lac-Promoter frei und bindet hochwirksam RNA-Polymerase, die ihrerseits DNA-Sequenzen, die nach dem Promoter folgen in mRNA "transcribiert". Die von der DNA-Sequenz codierten Proteine werden dann an der mRNA exprimiert, das heißt in Polypeptidketten "translatiert". Das Plasmid pGL101 enthält kurz nach dem lac-Promoter eine Schnittstelle für die Restriktionendonuclease PvuII, in die die Prä-S(1)-Sequenz eingesetzt werden soll.

Die Behandlung mit PvuII erfolgt in wässrigem Medium bei Temperaturen zwischen 34 und 40° C, vorzugsweise 36 und 38° C, bei pH-Werten zwischen 7,0 und 8,4, vorzugsweise 7,5 und 7,9; Zeit 15 Minuten bis 16 Stunden, vorzugsweise 1 bis 2 Stunden. Als Reaktionspuffer wird beispielsweise verwendet:
6 mM Tris-HCl; 60 mM NaCl; 6 mM $MgCl_2$; 7 mM beta-Mercaptoethanol.

...

Abstoppen der Reaktion durch Zugabe von 250 mM EDTA bis 10 mM Endkonzentration. Inaktivierung durch Erwärmen auf 65 - 80° C, vorzugsweise 65 - 70° C für 10 Minuten.

Plasmid pDR540 (Pharmacia-PL Biochemicals) enthält als Signalsequenz anstelle des lac uV5 Promoters, den tac Promoter, der im Prinzip genauso wirkt, jedoch eine etwa 10 mal stärkere Transcription der nachfolgenden DNA-Abschnitte bewirkt (Proc. Nat. Acad. Sci. USA 80 (1983) 21). Dieses Plasmid enthält kurz nach dem tac-Promoter eine BamHI Schnittstelle, in die die Prä-S(1)-Sequenz ebenfalls eingesetzt wurde.
Die Behandlung mit BamHI erfolgt in wässrigem Medium bei Temperaturen von 34 bis 40° C, vorzugsweise 36 und 38° C, bei pH-Werten zwischen 7,0 und 9,0, vorzugsweise 7,9 und 8,1; Zeit 15 Minuten bis 16 Stunden, vorzugsweise 1 bis 2 Stunden. Als Reaktionspuffer wird beispielsweise verwendet: 10 mM Tris-HCl; 100 mM NaCl; 5 mM $MgCl_2$, 1 mM beta-Mercaptoethanol.

Da BamHI Einzelstrangenden erzeugt, müssen diese noch mit Mung Bean Nuclease oder Enzymen ähnlicher Wirkung, wie vorher beschrieben, entfernt werden.

Die Kombination der aufgeschnittenen Plasmide pGL101 oder pDR540 mit dem Prä-S(1) DNA-Fragment erfolgt mit DNA-Ligase, insbesondere T4-DNA Ligase, in Gegenwart von 0,5 bis 2 mM Adenosintriphosphat (ATP), vorzugsweise 0,8 bis 1,2 mM in wässrigem Medium bei Temperaturen von 0 bis 20° C, vorzugsweise 4 bis 5° C und pH-Werten von 7,0 bis 8,2, vorzugsweise 7,5 bis 7,7. Als Reaktionspuffer wurde beispielsweise verwendet: 66 mM Tris-HCl; 5 mM $MgCl_2$; 5 mM DTT; 1 mM ATP.

Die so erhaltene Plasmid-DNA in der Reaktionsmischung wird zum Beispiel nach der Methode von Hanahan, J.Mol. Biol. 166 (1983), 557 - 580 in bakterielle Wirtszellen

...

0180012

transferiert und die Zellen mit der Plasmid-DNA selektioniert. Als Wirtszellen kommen in Frage: Bakterien, bevorzugt E.coli Stämme, die keine Restriktions- oder Rekombinationsfähigkeit haben, wie zum Beispiel E.coli-Stamm JM101.

Hierzu wurden E.coli Bakterien in LB-Medium (1 % Bactotrypton, 1 % Hefeextrakt, 0,5 % NaCl autoklaviert 20 Minuten bei 120° C) bis zu einer optischen Dichte bei 600 Nanometer ($OD_{600}$) von 0,1 bis 0,15 kultiviert, dann wird $MgCL_2$ bis zu einer Endkonzentration von 20 mM zugefügt und weiter bis zu einer $OD_{600}$ von 0,55 bis 0,65 kultiviert. Dann wird die Kultur rasch auf 0° C abgekühlt, die Zellen abzentrifugiert und die Zellen dann in dem von Hanahan beschriebenen Transformationspuffer resuspendiert, in der Kälte mit 3 bis 4 % Dimethylsulfoxid (DMSO) behandelt und dann kurz schockgefroren. Nach Zugabe von weiteren 3 bis 4 % DMSO wird die einzuschleusende DNA, etwa 1 bis 4 µg/ml, zugefügt, erneut in der Kälte inkubiert und schockgefroren. Durch diesen Prozess soll die Aufnahme der DNA in die Zellen bewirkt werden. Schließlich werden die Zellen aufgetaut und in einem zehnfachen Volumen von LB-Medium 0,5 bis 2 Stunden bei 37° C kultiviert. Von diesem Gemisch werden nun Einzelklone hergestellt, indem 0,1 bis 0,2 ml davon auf einem halbfesten Nährboden in 8 cm großen Petrischalen ausgestrichen werden. Der Nährboden besteht aus LB-Medium, in dem 1,5 % Agar in der Hitze aufgelöst wurden (LB-Agar). Um nur solche Bakterienzellen wachsen zu lassen, die tatsächlich eine funktionsfähige Plasmid DNA aufgenommen haben, wurde dem LB-Medium für diesen Zweck das Antibiotikum Ampicillin zugesetzt bevorzugt in Endkonzentrationen von 20 bis 200, vorzugsweise 50 bis 150 µg/ml (LBA-Agar). Das rekombinierte Plasmid enthält ein Gen, das Ampicillin-Resistenz vermittelt.

...

Nach Bebrütung über Nacht bei 37° C werden einige Dutzend bis einige Hundert Bakterienkolonien auf der Agarschicht sichtbar.

Nur ein kleiner Teil der aufgenommenen Plasmide enthält die gewünschte rekombinierte Plasmid DNA mit der Prä-S(1)-DNA-Sequenz. Zu ihrer Erkennung werden nun mit LBA-Medium angefeuchtete Nitrocellulosemembranen mit 80 mm Durchmesser auf die Agarplatten mit den Klonen aufgelegt und so Replicas der Ausgangsplatte hergestellt. Die Nitrocellulosemembran wird nun auf eine neue Agarplatte gelegt und bebrütet, so daß in spiegelbildlicher Anordnung die Klone der Ausgangsplatte wieder wachsen. Auf dieser Membran werden nun nach bekannten Methoden die Bakterienzellen der Klone lysiert und die DNA in situ mit Alkali in Einzelstränge aufgetrennt. Diese Einzelstränge binden sich hierbei irreversibel an die Membran. Nun wird die Membran mit einer bekannten einzelsträngigen HBV-DNA inkubiert, wobei diese HBV-DNA vorher durch sogenannte Nick-Translation mit $^{32}$P markierten Nucleotidtriphosphaten radioaktiv gemacht wurde. Diejenigen Klone, welche Prä-S(1)-DNA eingebaut haben, werden bei diesem Test die $^{32}$P markierte HBV-DNA aufgrund ihrer homologen Basensequenzen binden. Die entsprechenden Stellen auf der Membran werden durch Schwärzung eines Röntgenfilms erkannt. Die entsprechenden Klone auf der Ausgangsplatte können dann identifiziert und vermehrt werden. Diese gesamte Technik der "in situ" Hybridisierung ist ausführlich in T.Maniatis, E.F. Fritsch, J.Sambrook, Molecular Cloning Cold Spring Harbor Laboratories Seite 312 bis 328 beschrieben.

Die in situ Hybridisierung kann nicht die Polarität der eingesetzten Prä-S(1)-Sequenz erkennen. Die mRNA kann nur von 5' in 3' Richtung synthetisiert werden, so daß nur die Hälfte der rekombinierten Plasmide, jene mit der

...

richtigen Polarität, zur Synthese von Prä-S(1)-Protein führen werden. Eine Reihe von Klonen mit der Prä-S(1)-Sequenz wurde daher einzeln in LBA Medium kultiviert und aus diesen nach bekannten Methoden Plasmid-DNA (siehe Maniatis et al, Molecular Cloning, Seite 92) isoliert und im Falle von pGL101 durch Schnitte mit den Restriktionsenzymen EcoRI und Sau96I charakterisiert. Die Prä-S(1)-Sequenz enthält etwa 100 Basen vom Beginn der Prä-S(1)-Sequenz entfernt eine Sau96I Stelle, der Vector pGL101 etwa 100 Basen vor der Insertionsstelle eine EcoRI-Stelle. Es entsteht also bei richtiger Polarität ein 200 Basenpaar-Fragment. Bei falscher Polarität sind die letzten 300 Basen des Prä-S(1)-Fragment mit dem lacuv 5 Promoter verknüpft, so daß ein 400 Basenpaar-Fragment vorliegt.

Im Falle des Plasmids pDR540 wurden Prä-S(1) Insertionen der richtigen Polarität durch Schnitte mit Hind III und BalI ermittelt, wobei die richtige Polarität zu einem 260 Basenpaar-Fragment führt, die falsche zu einem 360 Basenpaar-Fragment. Die Größe der Fragmente wurde durch Gelelektrophorese in 5 % Polyacrylamidgel wie vorher beschrieben ermittelt.

Die Klone, die rekombinierte Plasmid DNA mit der Prä-S(1)-Sequenz in der richtigen Polarität enthalten, sollten im Prinzip Prä-S(1) Protein der Formel I in Anspruch 8 synthetisieren, mit X = MetGlnTrpLeuProArgAlaPheArg, sofern keine unerkannten Veränderungen der DNA-Sequenz eingetreten sind.

Bei zwei Klonen pKG1 und pKG2 wurde die Synthese des Prä-S(1)-Protein eindeutig nachgewiesen. Plasmid pKG1 besteht aus dem Plasmid pGL101 und der Prä-S(1)-Sequenz, pKG2 ist vom Vector pDR540 abgeleitet. Beide Plasmide befinden sich in E.coli JM101 und sind als Stämme E.coli

...

JM101 pKG1 mit der Aufnahme-Nummer DSM 3079 und als E.coli JM101 pKG2 mit der Aufnahme-Nummer DSM 3080 bei der Sammelstelle "Deutsche Sammlung von Mikroorganismen (DSM), Griesebachstraße 8, D-3400 Göttingen, hinterlegt.

Für die Produktion des Prä-S(1)-Protein wird einer der Stämme in LB-Medium mit 0,5 mM bis 2 mM IPGT, bevorzugt 1 mM, bei 35 bis 39° C, bevorzugt bei 37° C kultiviert und kurz nach Erreichen der stationären Phase abzentrifugiert. Die gepackten Zellen werden danach mit dem Enzym Lysozym, das Bakterienzellwände auflöst, und mit einem nichtionischen Detergens wie beispielsweise Nonidet P40, behandelt. Die Behandlung erfolgt in wässrigem Medium bei Temperaturen zwischen 0 und 4° C mit 1 bis 10 mg/ml Lysozym, vorzugsweise 4 bis 6 mg/ml bei pH 7,2 bis 7,6 für 5 bis 15 Minuten, vorzugsweise 8 bis 12 Minuten. Als Puffer dient beispielsweise 10 mM Tris-HCl und 1 mM EDTA. Danach wird das Gemisch bei 12 000 bis 18 000 U/Minute für 10 bis 20 Minuten bei 4° C abzentrifugiert. Der Überstand enthält neben löslichen E.coli Proteinen Prä-S(1)-Protein.

Das Prä-S(1)-Protein kann durch Immunaffinitätschromatographie angereichert und gereinigt werden. Dafür wird der monoklonale Antikörper MA18/7 kovalent an einen festen Träger gebunden. Als Träger sind beispielsweise poröse Glasperlen oder Agarosegelperlen geeignet. Für die kovalente Kopplung können im Handel erhältliche Derivate beider Materialien verwendet werden, zum Beispiel Aminopropylsilan beschichtete Glasperlen mit 140 nm Porenweite (Fluka) oder Bromcyan aktivierte Sepharose 4B (Pharmacia-PL-Biochemicals). Die Aminogruppe der beschichteten Glasperlen wird mit wässriger Glutardialdehydlösung aktiviert und zwar mit 1 bis 20 %, vorzugsweise 4 bis 8 % bei 0 bis 30° C, vorzugsweise 20 bis 25° C,

...

und bei pH 7 bis 10, vorzugsweise pH 8 bis 9 für 10 bis 60 Minuten, vorzugsweise 20 bis 40 Minuten. Das aktivierte Trägermaterial wird mit niedrigmolekularen alkalischem Puffer gewaschen und mit einem Überschuß MA18/7 versetzt. MA18/7 wird vorzugsweise aus Ascitesflüssigkeit gewonnen, und zwar durch Fällung mit 17 % Natriumsulfat.

Das gefällte Immunglobulin wird in 0,01 M Natriumcarbonat pH 9 wieder aufgelöst und mit dem aktivierten Trägermaterial 2 bis 20 Stunden, vorzugsweise 4 Stunden bei 0 bis 20° C, vorzugsweise 4 bis 8° C geschüttelt. Dabei tritt die kovalente Kopplung ein. Im Falle des Glutardialdehyd aktivierten Trägers werden die entstandenen Bindungen mit der Struktur einer Schiff'schen Base durch Reduktion mit Natriumborhydrid stabilisiert. Dies geschieht durch Rühren mit 0,05 bis 0,2 % Natriumborhydrid für 1 bis 4 Stunden bei 0° C und pH 9 ($\pm$1). Danach wird der trägerfixierte Antikörper MA18/7 mit PBS-Puffer, dann mit 3M Natriumthiocyanat (NaSCN) Lösung und wieder mit PBS Puffer gewaschen.

Der Prä-S(1)-haltige Rohextrakt wird nun mit dem so hergestellten Immunadsorbens umgesetzt. Hierzu kann das Immunadsorbens in eine Chromatographiesäule gepackt werden und dann leitet man langsam den Rohextrakt durch diese Säule oder man fügt dem verdünnten Rohextrakt (1:5 bis 1:20 verdünnt in PBS) das Immunadsorbens zu und schüttelt. Die Bindung des Prä-S(1)-Proteins erfolgt bei 0 bis 40° C, vorzugsweise 18 bis 25° C im Verlauf von 0,5 bis 24 Stunden, vorzugsweise 1 bis 4 Stunden. Es schließen sich eine gründliche Waschung mit PBS, mit 0,5 bis 1,5 M NaCl-Lösung, bevorzugt 1 M NaCl und wieder mit PBS an, um E.coli-Proteine so gut wie möglich zu entfernen.

...

Die Ablösung des Prä-S(1)-Protein erfolgt mit 3 M NaSCN oder mit saurem Puffer von pH 1,8 bis 2,2, beispielsweise mit 0,2 M Citronensäurelösung. Hierzu befindet sich das Immunadsorbens in einer Chromatographiesäule und der Dissoziationspuffer wird durchgepumpt und das Eluat fraktionsweise aufgegangen. In den Fraktionen wird das abgelöste Protein durch die optische Dichte bei 280 Nanometer nachgewiesen. Der spezifische Nachweis von Prä-S(1) erfolgt durch Auftropfen von 5 µl jeder Fraktion auf eine poröse (1,2 µm Poren) Nylonmembran. Nach dem Eintrocknen der Tropfen wird die Membran zuerst in foetales Kalbserum zur Absättigung der unspezifischen Bindungen gelegt, danach wird MA 18/7 zu dem Kalbserum zugefügt und unter Schütteln inkubiert, dann wird mit PBS gewaschen und schließlich ein Antikörper gegen Maus-Immunglobulin zugefügt, der kovalent das Enzym Meerrettich-Peroxidase gebunden enthält. Dieses Peroxidase markierte anti-Maus Ig ist im Handel erhältlich. Es wird 1:100 bis 1:2000, vorzugsweise 1:200 in 20 % foetalem Kalbserum zur Membran gegeben. Dort wo die Membran Prä-S(1)-Protein aus der Probe adsorbiert hat, bindet sich auch MA18/7 und später Peroxidase. Die gebundene Peroxidase wird mit einem bekannten Farbtest nachgewiesen. Das Enzym oxidiert Diaminobenzidin in Gegenwart von $H_2O_2$ zu einem unlöslichen braunen Farbstoff. Die Intensität der braunen Flecke ist ein semiquantitatives Maß für die Menge des vorliegenden Prä-S(1)-Proteins.

Die Fraktionen, die in dem besagten Test eine starke Reaktion ergeben, werden vereinigt und in einem Dialysierschlauch oder einer Ultrafiltrationshülse (zum Beispiel der Kolloidhülse der Firma Sartorius) gegen PBS dialysiert und bis zur weiteren Verwendung eingefroren, wenn diese nicht innerhalb von einigen Tagen erfolgt.

...

Zu dem Verfahren b):

Es werden Hepatitis B-Viruspartikel oder Hepatitis B-Oberflächenantigen eingesetzt. Diese Ausgangsmaterialien werden vorher auf bekannte Weise gereinigt. Das Verfahren erfolgt bei Temperaturen zwischen 0 - 100° C, vorzugsweise 20 - 50° C in wässrigen Medium.
Die Behandlung mit Bromcyan erfolgt vorzugsweise bei Temperaturen zwischen 0 - 50° C in wässrigem Medium bei pH-Werten unter 7. Der saure pH-Wert kann eingestellt werden mit anorganischen Säuren (zum Beispiel Salzsäure, Schwefelsäure, Phosphorsäure) oder organischen Säuren. Als organische Säuren kommen zum Beispiel solche mit $P_K$-Werten zwischen 3 und 4,9 in Frage (zum Beispiel Ameisensäure, Essigsäure). Das Bromcyan wird im Überschuß verwendet, beispielsweise mindestens 2facher bis maximal 5facher Überschuß (bezogen auf Gewicht). Bei der Behandlung mit Bromcyan erhält man Peptide, die im wesentlichen aus der Prä-S(1)-Aminosäuresequenz bestehen sowie gegebenenfalls auch Fragmente von solchen Peptiden.

Die Behandlung mit dem Detergenz erfolgt vorzugsweise zwischen 20 - 100° C, insbesondere 35 bis 40° C während 5 bis 30 Minuten, vorzugsweise 5 bis 10 Minuten in wässrigem Medium. Als Detergentien kommen zum Beispiel in Frage: lösliche Salze von aliphatischen Schwefelsäuremonoestern, zum Beispiel Natriumlaurylsulfat.
Die Konzentration der Detergentien in dem wässrigen Medium ist zum Beispiel 0,1 bis 20 %, vorzugsweise 0,5 bis 3 %. Als weitere Zusatzstoffe bei dieser Detergenzbehandlung kommen noch in Frage: Reagenzien, welche Disulfidbrücken spalten, das heißt Verbindungen mit freien SH-Gruppen, wie organische Sulfhydrylverbindungen, beispielsweise Dithiothreit, Dithioerythrit,

...

2-Mercapto-ethanol. Man erhält durch die vorstehend angegebene Detergenz-Behandlung dissoziierte Virus-Proteine. Die so erhaltene Proteinmischung enthält unter anderem 2 Proteine mit dem Molgewicht 39 000 und 42 000 Dalton (Protein P39 und Protein GP42), die durch Gelelektrophorese in bekannter Weise isoliert werden können.

Die durch die Detergenz-Behandlung erhaltene Peptid-Mischung kann durch anschließende Behandlung mit Protease noch weiter in kleinere Peptide mit der Prä-S(1)-Sequenz zerlegt werden.

Als Protease kommen zum Beispiel in Betracht: V8 Protease aus Staphylococcus aureus. Diese Protease spaltet Polypeptidketten bevorzugt an Stellen mit Glutaminsäure. Diese Reaktion findet bei Temperaturen von 20 bis 50° C, vorzugsweise 35 bis 40° C bei pH 6,0 bis 8,0, vorzugsweise 7,2 bis 7,6 im Verlauf von 1 bis 4 Tagen statt. Die Reaktionsmischung soll nicht mehr als 2 % Natriumlaurylsulfat und 2 % Dithiothreitol enthalten, vorzugsweise jeweils 0,5 bis 1,2 %. Das abgespaltene Protein mit der Prä-S(1)-Sequenz läßt sich aus dem Gemisch beispielsweise durch Gelelektrophorese abtrennen. Hierfür wird Polyacrylamidgel mit 10 bis 18 % Polymergehalt, vorzugsweise 13 bis 15 % eingesetzt. Das Gel hat die Form von flachen Schichten mit eingeformten Probentaschen. Das Puffersystem ist von Laemmli angegeben worden (Nature 227, 1970, 680-685), weitere Angaben zur Ausführung der Elektrophorese und zur Isolierung der aufgetrennten Proteine finden sich in "Virology" 123, 1982, 436-442. Nach beendigter Elektrophorese befinden sich die Proteine je nach ihrer Größe in einer bestimmten Zone des flachen Gels. Die Zone, wo sich das Prä-S(1)-Protein befindet, läßt sich durch

...

Vergleich mit bekannten Vergleichproteinen bestimmen, wobei diese Proteine und die Proteine des Probengemisches am besten durch eine Färbung mit Silber sichtbar gemacht werden. Die Zone mit dem 18 000 Dalton großen Proteinfragment wird ausgeschnitten, zerkleinert und mit Pufferlösung eluiert.

Durch die anschließende Behandlung mit Protease erhält man Peptide, die im wesentlichen aus der Prä-S(1)- und Prä-S(2)-Aminosäuresequenz bestehen.

...

Zu dem Verfahren c):

Dieses Verfahren erfolgt durch Synthese aus den entsprechenden, gegebenenfalls in üblicher Weise geschützten Aminosäuren, in herkömmlicher Weise. Ebenfalls ist die Verwendung automatischer Peptid-Synthesierer, zum Beispiel Beckman Model 990 Peptid Synthetisierer möglich unter Verwendung der auf übliche Weise im Handel erhältlichen geschützten Aminosäuren.

Diese Synthese von beispielsweise Peptiden gemäß Anspruch 7 oder 8 erfolgt zum Beispiel in der hierfür üblichen Weise aus den entsprechenden geschützten Aminosäuren, indem man zunächst die Carboxy-terminale Aminosäure des zu synthetisierenden Peptids, deren $\alpha$-ständige Aminogruppe geschützt ist, an einen hierfür üblichen synthetischen Träger kovalent bindet, die $\alpha$-Amino-Schutzgruppe abspaltet, an die so erhaltene freie Aminogruppe die nächstfolgende geschützte Aminosäure über ihre Carboxy-Gruppe bindet, dann wiederum die $\alpha$-Amino-Schutzgruppe dieser zweiten Aminosäure abspaltet, hieran die nächste Aminosäure bindet und in dieser Weise Schritt für Schritt die übrigen Aminosäuren des zu synthetisierenden Peptids in der richtigen Reihenfolge verknüpft und nach Verknüpfung aller Aminosäuren das fertige Peptid vom Träger abspaltet und gegebenenfalls weitere vorhandene Seitenfunktions-Schutzgruppen abspaltet.

Die Reaktionen zur Verknüpfung der Aminosäuren finden in dem Temperaturbereich von 10 - 40° C, vorzugsweise 20 - 30° C, gegebenenfalls in einem hierfür üblichen indifferenten Lösungs- oder Suspensionsmittel (zum Beispiel Dichlormethan) statt, wobei gegebenenfalls zur Verbesserung der Löslichkeit bis zu 20 % Dimethylformamid zugesetzt werden kann.

Als synthetisches Trägermaterial kommen synthetische Polymere in Frage, zum Beispiel quellbares Polystyrolharz in Perlenform (beispielsweise ein chlormethyliertes

...

Copolymerisat aus Polystyrol und 1 % Divinylbenzol).
Als Schutzgruppen für die $\alpha$-ständigen Aminogruppen
kommen zum Beispiel in Frage: tertiäre Butyloxycarbonylgruppe, Carbobenzoxygruppe beziehungsweise Carbobenzthiogruppe (gegebenenfalls jeweils mit p-Brom- oder
p-Nitro-benzylrest), Trifluoracetylgruppe, Phthylrest,
o-Nitrophenoxy-acetylgruppe, Tritylgruppe, p-Toluolsulfonylgruppe, Benzylgruppe, im Benzolkern substituierte Benzylreste (p-Brom- oder p-Nitro-benzylrest), $\alpha$-Phenyl-ethyl-
rest. Hierzu wird auch auf das Buch von Jesse P. Greenstein und Milton Winitz, Chemistry of Amino Acids, New
York 1961, John Wiley and Sons, Inc., Volume 2, beispielsweise Seite 883 und folgende sowie The Peptids,
Volume 2, Special Methods in Peptide Synthesis, Part A,
Ed. E. Gross and J. Meienhofer, Academic Press, New York,
1980, Tabelle III, Seite 20 und Tabelle IV, Seite 21
verwiesen. Diese Schutzgruppen kommen grundsätzlich auch
für den Schutz von weiteren funktionellen Seitengruppen
(OH-Gruppen, NH$_2$-Gruppen) der in Frage kommenden Aminosäuren in Frage.
Vorhandene Hydroxygruppen (Serin, Threonin) werden vorzugsweise durch Benzylgruppen und ähnliche Gruppen geschützt. Weitere nicht $\alpha$-ständige Aminogruppen (zum Beispiel Aminogruppen in $\omega$-Stellung; Guanidinogruppe des
Arginins) werden vorzugsweise mit Nitro-Gruppen geschützt.

Die Verknüpfung der einzelnen Aminosäuren miteinander
erfolgt nach den hierfür üblichen Methoden. Insbesondere
kommen in Frage: Methode der symmetrischen Anhydride (in
Gegenwart von Dicyclohexylcarbodiimid),
Carbodiimid-Methode,
Carbodiimid-Hydroxybenzotriazol-Methode.
Als weitere Methoden kommen zum Beispiel in Frage:
Azid-Methode; Mischanhydrid-Methode; N-Carbonsäureanhydrid-
Methode; Methode der aktivierten Ester; Verwendung von
modifizierten Carbodiimiden.

...

Die angegebenen Peptidsynthese-Methoden sind beispielsweise in dem Buch: Aminosäuren, Peptide, Proteine, Ed. H.-D. Jakubke, H. Jeschkeit, Verlag Chemie, Weinheim, 1982 beschrieben.

Für die Verknüpfung von Arginin wird vorzugsweise die Carbodiimid-Methode benutzt, für die Verknüpfung von Asparagin sowie von Glutamin wird vorzugsweise die Carbodiimid-Hydroxy-benzotriazol-Methode benutzt (siehe The Peptids, Volume 2, Ed. E. Gross and J. Meienhofer). Für die übrigen Aminosäuren wird im allgemeinen die Methode der symmetrischen oder gemischten Anhydride benutzt.

Beispielhaft ist die Synthese für das Peptid Cc beziehungsweise Cd (Seite 24) im folgenden angegeben): Die Sequenz dieser Oligopeptide ist in der Figur 6 dargestellt. Zur Synthese wurden N$\alpha$-tertiär-Butyl-oxycarbonyl (N$\alpha$-BOC) geschützte L-Aminosäuren (Fluka) verwendet. Die Seitengruppenfunktionen des L-Serin und L-Threonin waren zusätzlich mit O-Benzyl-Gruppen, die des L-Arginin mit N$\omega$-Nitro-Gruppen geschützt. Zuerst wurde N$\alpha$-BOC-L-Prolin über eine 4-Oxymethylphenylacetat-Verbindung (J.Am.Chem.Soc. 98 (1976) 7357-7362; J.Org.Chem. 43 (1978) 2845-2852) an das Trägermaterial Biobeads S-X1 (Bio-Rad) gebunden (BOC-Pro-PAM-Polystyrol). Nach Abspaltung der N-terminalen BOC-Schutzgruppe durch Säure (Nature 206 (1965) 619-620) wird die nächste N$\alpha$-BOC-geschützte Aminosäure gekoppelt. Bei diesen Kupplungsschritten wurden 3 verschiedene Verfahren angewendet. N$\alpha$-BOC-N$\omega$-Nitro-L-Arginin wurde unter Anwendung von Dicyclohexyl-Carbodiimid (DCC; J.Am.Chem. Soc. 77 (1955) 1067-1068; J.Am.Chem.Soc. 88 (1966) 1013-1030), N$\alpha$-BOC-L-Asparagin und L-Glutamin unter Anwendung von Dicyclohexylcarbodiimid/1-Hydroxybenzotriazol (DCC/HOBT; Chem.Ber. 103 (1970) 788-798; J.Org.Chem. 45 (1980) 555-560) und die restlichen geschützten

...

Aminosäuren über symmetrische Anhydride gebunden (SA; J.Am.Chem.Soc. 97 (1975) 6584-6585; Angew. Chem. Int. Ed. Engl. 10 (1971) 336; Hoppe-Seyler's Z.Physiol. Chem. 353 (1972) 1973-1976). Nach Kupplung der 10. Aminosäure L-Asparagin wurde das Trägermaterial geteilt und an die eine Hälfte N -BOC-L-Alanin gebunden. Die Synthesen wurden getrennt bis zur 16. Aminosäure weitergeführt und getrennt gespalten. Die Abspaltung der fertigen Peptide vom Trägermaterial unter gleichzeitiger Abspaltung der Seitenfunktions-Schutzgruppen wurde mit Hydrogenfluorid und 10 % (v/v) Anisol durchgeführt (Bull.Chem.Soc.Jpg. 40 (1967) 2164-2167; J.Org.Chem. 35 (1970) 3151-3152; J.Am.Chem.Soc. 97 (1975) 3485-3496).

Der Prä-S(1)-Anteil der Proteine P39/GP42 befindet sich auf der Oberfläche des HBV-Partikels. Immunisiert man Mäuse mit HBV-Partikeln, bilden sie Antikörper gegen Prä-S(1)-Polypeptide. Aus den Milzzellen einer so immunisierten Maus wurde nach bekannten Methoden (J.Immunol. 23; 1548-1550) (1979)) eine Hybridomzell-Linie erzeugt, die einen monoklonalen Antikörper (MA18/7) gegen Prä-S(1)-Polypeptid produziert. Die Zell-Linie und der entsprechende Antikörper werden sowohl in vitro als Zellkultur als auch in vivo als Ascites-Tumor in BALB/c Mäusen weiter vermehrt. MA18/7-Antikörper reagieren gut mit HBV-Partikeln der Subtypen $adw_2$ oder ayw, mit HBsAg Filamenten und in etwa 5 bis 20 mal geringer Bindungsstärke pro Antigenmenge mit 20 nm HBsAg-Partikeln. Isoliertes P39/GP42 bindet ebenfalls MA18/7. Spaltet man die Prä-S-Sequenz aus P39/GP42 mit einer Glutaminsäure spezifischen Protease, so entsteht ein aminoterminales Polypeptidfragment von 164 beziehungsweise 175 Aminosäuren das ebenfalls MA18/7 bindet. Die Spaltstelle befindet sich 1 Codon nach dem Aminoende des HBsAg Hauptproteins. Durch diese Behandlung läßt sich aus HBV, Filamente und HBsAg 20 nm Partikel ein Polypeptid erzeugen, das die wichtigsten virusspezifischen Antigendeterminanten enthält.

...

Die Erfindung betrifft daher auch den monoklonalen Antikörper MA18/7 beziehungsweise die Hybridzell-Linie Go 1 A 18/7-1. Dieser Antikörper MA18/7 besitzt eine hohe Spezifität für Peptide, die die Prä-S(1)-Aminosäuresequenz enthalten. Der neue Antikörper MA18/7 ist insbesondere zur Hochreinigung und zum Nachweis von Peptiden mit der Prä-S(1)-Sequenz geeignet.

Erfindungsgemäß erhält man die neuen Immunglobulinproduzierenden Hybridzell-Linien durch Zellfusion.

Hierzu werden Milzzellen, die unmittelbar nach ihrer Entnahme aus den immunisierten Mäusen (zum Beispiel Stamm BALB/c) mit Myelomzellen, zum Beispiel Myelomzellen X63-Ag8-653 genau nach der Methode, die in der Broschüre Hybridoma Techniques, Verlag Cold Spring Harbor Laboratory 1980, Cold Spring Harbor, Staat New York, USA hierfür angegeben ist fusioniert, anschließend kloniert (unter Bedingungen der limitierenden Verdünnung), selektioniert und kultiviert. X63-Ag8-653 Myelom-Zellen sind ein Subklon aus dem P3-X63-Ag8 Myelom-Subklon, siehe Kearney, J. et al (1979), Journal of Immunology 123, No. 4, 1548.

Die Kulturen, welche Hybridzellwachstum zeigen, werden auf den Gehalt an Prä-S(1)-spezifischen Antikörpern auf folgende Weise geprüft:

Aus Kulturüberständen von Näpfen, in denen das Hybridzellwachstum durch Bildung von Zellhaufen erkennbar wurde, werden Aliquote in Mikrotiternäpfe gegeben, die mit verdünnten HBV-Partikeln beschichtet waren (die HBV-Partikel waren mit Natriumphosphat-Lösung vom pH 7,4 verdünnt). Durch Zugabe von bekanntem anti-Maus Immunglobulin, das durch Peroxidase markiert war, wird in bekannter Weise festgestellt (Farbreaktion), ob sich Antikörper aus den entsprechenden Kulturen an das HBV-Antigen gebunden haben (siehe die erwähnte

...

Broschüre Hybridoma Techniques). Die Proben, die eine kräftige Farbreaktion zeigen, werden dann in größere Kulturgefäße gegeben und darauf geprüft, ob sie mit Prä-S(1)-Polypeptiden (zum Beispiel den Peptiden P39 beziehungsweise GP42 reagieren, hingegen nicht mit dem in Virology Band 123 (1982), Seiten 436 - 442 beschriebenen Peptid GP33. Die Kulturen, die diese Voraussetzungen erfüllen, werden noch einmal unter den Bedingungen der limitierenden Verdünnung kloniert. Die so erhaltenen Klone produzieren monoklonale Antikörper gegen Prä-S(1)-Peptide. Einer der so erhaltenen Antikörper wird als MA18/7 bezeichnet und in üblicher Weise als Zellkultur vermehrt. Diese Vermehrung erfolgt zum Beispiel in Flaschen in RPMI Earle 199/10 % foetalem Kalbserum, wobei die Innenwand der Flaschen pro cm² mit $10^3$ peritonealen Macrophagen der Maus (Stamm NMRI) bedeckt ist. Die Kulturen werden bei 37° C und 5 % $CO_2$/Luft gehalten. Die Vermehrung kann aber zum Beispiel auch erfolgen als Ascitestumor in syngenen Mäusen, zum Beispiel BALB/c Mäusen. Hierzu werden den Mäusen 10 Tage vorher 0,5 ml Pristan (2,6,10,13-Tetramethyl-pentadecan) intraperitoneal injiziert, dann werden 3 x $10^6$ der entsprechenden Hybridomzellen injiziert.

Die Isolierung des neuen monoklonalen Antikörpers aus der Ascitesflüssigkeit der tumortragenden Mäuse erfolgt nach bekannten Methoden, zum Beispiel durch fraktionierte Fällung vorzugsweise mit Natriumsulfat oder Ammoniumsulfat und gegebenenfalls anschließender Chromatographie über einen geeigneten Träger, vorzugsweise auf Zellulose-Basis, wie zum Beispiel Diethylaminoethyl-Zellulose. Die Isolierung aus Hybridomüberstand (Zellkulturüberstand) erfolgt in bekannter Weise, vorzugsweise durch Bindung an Sepharose (perlförmiges Agarose-Gel), an welche Staphylococcen-Protein A gekoppelt ist und anschließende

...

- 44 -

Ablösung mittels einer wässrigen Pufferlösung.

Der so hergestellte Antikörper kann nun direkt zum Nachweis für Prä-S(1)-Peptide beziehungsweise nach seiner kovalenten Bindung an einen biologisch inaktiven Träger zur Hochreinigung von Prä-S(1)-Peptiden verwendet werden. Die kovalente Bindung des Antikörpers erfolgt hierbei an einen entsprechend aktivierten Träger, vorzugsweise auf Agarose-Basis (wobei die Agarose zum Beispiel mit Bromcyan aktiviert ist) oder an porösen Glasperlen, beispielsweise an Aminopropylgruppen enthaltenden Glasperlen (Firma Fluka/Schweiz). Zur Hochreinigung wird zum Beispiel eine Lösung des Prä-S(1)-Peptids bei schwach basischen pH, zum Beispiel bei einem pH 7 - 9 über einen so hergestellten Antikörper-Affinitätsträger gepumpt, solange bei neutralem pH gewaschen, bis das Eluat proteinfrei ist, und anschließend das gebundene Peptid mit der Prä-S(1)-Aminosäuresequenz mit konzentrierten Lösungen von chaotropen Ionen beispielsweise hochmolarer (2 molar bis Sättigung, insbesondere 3 molar) Alkalithiocyanat-Lösung, beispielsweise Natriumthiocyanat-Lösung bei pH 7 oder mit sauren Pufferlösungen zwischen pH 1,6 - 2,5 eluiert. Die so erhaltenen proteinhaltigen Fraktionen können beispielsweise vom Eluierungsmittel durch Dialyse bei pH 7 oder Gelfiltration (zum Beispiel Sephadex G25) befreit werden, wobei diese Maßnahmen unter Verwendung von physiologischen Puffern durchgeführt werden.

Die Zell-Linie Go 1 A 18/7-1 ist unter der Nr. 84101501 bei folgender Sammelstelle hinterlegt: Public Health Laboratory Service, National Collection of Animal Cell Cultures, Porton Down, Salisbury, Wiltshire SP40JG, Großbritannien.

Diese Zell-Linie produziert den monoklonalen Antikörper MA 18/7, der bezüglich seiner konstanten Merkmale zur

...

Immunoglobulin-Klasse $IgG_1$/kappa gehört. Der Antikörper verbindet sich mit der Prä-S(1)-Aminosäuresequenz von Proteinen oder/beziehungsweise mit Proteinen, die Fragmente dieser Prä-S(1)-Aminosäuresequenz enthalten, wobei solche mindestens 6 Aminosäuren enthalten müssen.

Die Hybridzellen Go 1 A18/7-1 können gefroren in flüssigem Stickstoff aufbewahrt werden, in dem weiter unten beschriebenen Kulturmedium, welches zusätzlich 10 % Dimethylsulfoxid enthält. Die Einfrierrate sollte langsam sein, etwa 1 - 2° C/Minute. Zum Zwecke der weiteren Vermehrung werden die gefrorenen Zellen beispielsweise (Minitubes mit $3 \times 10^6$ gefrorenen Zellen) schnell aufgetaut und langsam (innerhalb von 10 Minuten) mit 10 ml Kulturmedium vermischt, welches beispielsweise die folgende Zusammensetzung hat:

| | |
|---|---|
| 24,35 g | Medium 199/Earle (siehe Morgan, J.F. et al., Proc. Soc. Exp. Biol. Med. 73, Seite 1, 1950) |
| 26,00 g | RPMI/1640 (siehe Moore, G.E. et al., J. Am. Med. Assoc. 199, Seite 519, 1967; Earle, W.R. et al, J. Nat. Cancer Inst. 4, Seite 165, 1943) |
| 10,00 g | $NaHCO_3$ |
| 50 ml | Glutamin 200 mM |
| 50 ml | Nicht-essentielle Aminosäuren (Mischung der üblichen nicht-essentiellen Aminosäuren) |
| 50 ml | Antibiotikum-Antimycotikum-Lösung |
| 50 ml | Natriumsalz der Brenztraubensäure 100 mM in MEM (minimales essentielles Medium) |
| 500 ml | foetales Kalbserum |
| ad 5 Liter | |

mit bidestilliertem Wasser und mit $CO_2$ auf pH 6,9 - 7,0 gebracht und durch Filtration sterilisiert.

Auch das folgende Kulturmedium kann beispielsweise verwendet werden:

...

Mixed-Medium 80/20 aus

80 % RPMI 1640

20 % Medium 199/Earle

zusätzlich: 20 g $NaHCO_3$

100 ml 200 mM L-Glutamin

100 ml 100x nicht-essentielle Aminosäuren

100 ml 100x Antibiotikum-Antimycoticum-

Lösung: 10 000 Einheiten Penicillin 10 000 µg Streptomycin 25 µg Fungizone

100 ml 100 mM Natriumpyruvat

1 Liter FCS (fötales Kälberserum)

auf 10 Liter mit bidestilliertem Wasser

auffüllen, mit $CO_2$ auf pH 6,7-7,0 einstellen und durch Filtration sterilisieren.

Anschließend werden die Zellen zentrifugiert (500fache der Erdbeschleunigung), die sedimentierten Zellen in 30 ml des zuvor angegebenen Mediums resuspendiert und anschließend in Zellkulturflaschen mit 75 cm² Bodenfläche gegeben.

Polypeptide mit der Prä-S(1)-Sequenz, insbesondere solche, die im wesentlichen aus der Prä-S(1)-Sequenz bestehen, oder aus Untereinheiten dieser Prä-S(1)-Sequenz, sind als Impfstoffe gegen Hepatitis B-Virus-Erkrankungen geeignet. Solche Impfstoffe enthalten als Wirkstoffe mindestens 1 erfindungsgemäßes Peptid (zum Beispiel ein Peptid gemäß Anspruch 8). Der Impfstoff kann aber auch 2 oder mehr der erfindungsgemäßen Peptide (zum Beispiel gemäß Anspruch 8) enthalten. Vorzugsweise handelt es sich um Impfstoffe, die als Wirkstoff mindestens ein Peptid der Formel I mit den angegebenen Bedeutungen für X enthalten und gegebenenfalls zusätzlich noch ein Peptid der Formel IV mit den angegebenen Bedeutungen für X. Vorzugsweise hat X hierbei die Bedeutung: MetGlnTrpLeuProArgAlaPheArg-OH.

...

Das Gemisch dieser beiden Peptide hat den Vorteil, daß hier neben den Antikörpern gegen alle Virussubtypen auch die Mehrzahl der subtyp-spezifischen Antikörper gegen die Prä-S(1)-Sequenz induziert wird.

Die Anwendung der erfindungsgemäß erhaltenen Polypeptide oder Oligopeptide zur Bekämpfung von Hepatitis B-Erkrankungen erfolgt in der für Impfstoffe üblichen Art beispielsweise durch Injektion oder in Form von Sprays.

Insbesondere bewirken die erfindungsgemäßen Prä-S(1)-Peptide eine Immunisierung gegenüber Hepatitis B-Virus-Partikeln, kaum dagegen gegen die ungefährlichen HBsAg 20 nm Partikel.

Die erfindungsgemäß erhaltenen Prä-S(1)-Peptide werden zur Herstellung von Impfstoffen durch Immunaffinitätschromatographie und die üblichen Verfahren gereinigt (Filtration, insbesondere Molekularfiltration und Gelfiltration, Separation, Gradientenzentrifugation, Adsorption und anschließende Elution usw.), gegebenenfalls konzentriert (Zonenzentrifugation, Molekularfiltration, Adsorption und anschließende Elution), durch Hinzufügen von Stabilisatoren stabilisiert und mit geeigneten Konservantien (zum Beispiel Benzylalkohol, Chlorbutanol, Thiomersal, Phenol, Kresol) haltbar gemacht.

Weiterhin kann die Antigenität der Impfstoffe durch Hinzufügen von Adjuvantien verstärkt beziehungsweise eine Depotwirkung erzielt werden.

Dazu kommen beispielsweise Freund'sches Adjuvans, ölige und andere organische Adjuvantien insbesondere jedoch Aluminiumverbindungen wie Aluminiumoxid, Aluminiumhydroxid und Aluminiumphosphat in Frage.

Die Prä-S(1)-Peptide können ebenfalls als Bestandteil von Kombinationsimpfstoffen verwendet werden.

...

Beschreibung der hinterlegten Mikroorganismen, die bei der Deutschen Sammlung von Mikroorganismen (DSM), Griesebachstraße 8, D-3400 Göttingen gemäß dem Budapester Abkommen hinterlegt sind. Es handelt sich um folgende Mikroorganismen (Reinkulturen):

1) E.coli K490 pHBV991 mit der Hinterlegungsnummer DSM 3082.
   Der Mikroorganismus E.coli (Escherichia coli) K490 pHBV991 produziert den Klon pHBV991. Es handelt sich um das Bacterium Escherichia coli K12 Stamm 490, welches das Plasmid pHBV991 enthält (Reinkultur). Das Plasmid besteht aus dem Ausgangsplasmid pBR322 und Hepatitis B-Virus-DNA (Isolat F991), die in die BamHI-Stelle insertiert ist. Das Plasmid exprimiert kein virales Genprodukt.

2) E.coli JM101 pGL101 mit der Hinterlegungsnummer DSM 3081.
   Dieser Mikroorganismus ist ein Escherichia coli K12 Abkömmling (Messing et al, 1981, Nucleic Acids Res. 9:309) mit Plasmid pGl101. Das Plasmid ist eine rekombinante DNA aus dem Origin und der ß-Lactamase von pBR322, aus dem tac-promoter und aus einem Prä-s(1)-Fragment der Hepatitis B-Virus-DNA. Es exprimiert Ampicillinresistenz.

3) E.coli JM101 pKG1 mit der Hinterlegungsnummer DSM 3079.
   Dieser Mikroorganismus ist ein Escherichia coli K12 Abkömmling (Messing et al, 1981, Nucleic Acids Res. 9:309) mit Plasmid pKG1. Das Plasmid ist eine rekombinante DNA aus dem Origin und der ß-Lactamase

...

von pBR322, aus dem lacuv5-Promoter und aus einem Prä-s(1)-Fragment der Hepatitis B-Virus-DNA. Ex exprimiert Ampicillinresistenz und ein Prä-s(1)-Polypeptid mit 119 Aminosäuren.

4) E.coli JM101 pKG2 mit der Hinterlegungsnummer DSM 3080. Dieser Mikroorganismus ist ein Escherichia coli K12 Abkömmling (Messing et al, 1981, Nucleic Acids Res. 3:309) mit Plasmid pKG2. Das Plasmid ist eine rekombinante DNA aus dem Origin und der ß-Lactamase von pBR322, aus dem tac-Promoter und aus einem Prä-s(1)-Fragment der Hepatitis B-Virus-DNA. Es exprimiert Ampicillinresistenz und ein Prä-s(1)-Polypeptid mit 119 Aminosäuren.

Die Züchtungs- und Aufbewahrungsbedingungen für die vorstehend unter 1) bis 4) genannten Mikroorganismen sind die folgenden:

Medium:
Luria Broth: Hefeextrakt Difco 10 g, Bactotrypton Difco 10 g, NaCl 5 g, pH mit NaOH einstellen, auf 1 l mit dest. $H_2O$, 100 mg Ampicillin (Binotal, Bayer) in steriler Lösung zufügen, pH vor Sterilisation: 7,5, Sterilisation 20 Minuten bei 120° C, Verhalten gegenüber Sauerstoff: aerob, Bebrütungstemperatur: 37° C, Bebrütungsdauer: über Nacht, Aufbewahrung bei: 4° C, Überimpfungsintervall: 3 Monate.

Aufbewahrungsbedingungen: 4° C auf Agar oder -20° C in 50 % Glycerin-LB-Ampicillin. Bedingungen für die Lebensfähigkeitsprüfung: Ausstreichen auf Agar mit dem oben angegebenen Luria Broth-Medium.

...

Beispiele

Soweit nicht besonders angegeben, werden in den folgenden Beispielen allgemein erwähnte Verfahrensschritte, wie zum Beispiel enzymatische Reaktionen, Präzipitieren, Reinigungen usw. gemäß den Anleitungen des Buches Molecular Cloning, A Laboratory Manual, von T. Maniatis, E.F. Fritsch, J. Sambrook, Cold Spring Harbor Laboratories, 1982, durchgeführt.

Im folgenden bedeutet stets:

M     : wässrige Lösung, die pro Liter ein Mol der betreffenden Substanz enthält,

mM    : wässrige Lösung, die pro Liter ein Millimol der betreffenden Substanz enthält,

µM:   : wässrige Lösung, die pro Liter ein Mikromol der betreffenden Substanz enthält.

Beispiel I:

1.   Herstellung der Ausgangs-DNA pHBV991:

Isolierung der HBV-Partikel (HBV = Hepatitis B-Virus)

Als Rohstoff für die Gewinnung der HBV-DNA werden zunächst HBsAg positive Blutkonserven gesammelt, die von Blutspendediensten ausgesondert werden. Die Proben werden auf HBsAg mit der quantitativen Immunelektrophorese

...

(Develop. Biol. Standard 30, 1975, 78-87) und auf HBeAg mit einem handelsüblichen Radioimmunassay (RIA) untersucht. Proben mit mehr als 25 µg HBsAg/ml und mit einem HBeAg-Titer über 1:100 enthalten mit großer Wahrscheinlichkeit mehr als $10^8$ HBV-Partikel/ml und sind als Startmaterial geeignet. Etwa 5 % der HBsAg positiven Blutspender haben solche Werte. Natürlich kann von solchen Blutspendern oder bekannten HBV-Trägern gezielt Blutplasma durch Plasmaphorese gesammelt werden. Als Startmenge werden mindestens 10 ml, besser aber 40 bis 70 ml Plasma verwendet.

Das Plasma wird zunächst bei 10 000 U/Minute und 4° C in einem Winkelrotor zentrifugiert. Sediment und Fettschicht werden verworfen, das geklärte Plasma mit einer Pipette abgesaugt. Je 10 ml des geklärten Plasmas werden in einem Ultrazentrifugenröhrchen der Maße 14 x 95 mm auf einen diskontinuierlichen Saccharosegradienten bestehend aus 2 ml 30 %iger (Gewicht/Volumen) und 0,5 ml 20 %iger Saccharoselösung geschichtet. Die Lösung ist in 130 mM NaCl, 20 mM Tris-HCl pH 7,4 angesetzt. Je nach verfügbarer Plasmamenge werden bis zu sechs Zentrifugenröhrchen auf diese Weise gefüllt und 4 Stunden bei 38 000 U/Minute und 10° C in einem Schwingbecherrotor ultrazentrifugiert. Der erhaltende Überstand wird einschließlich der Lipide vollständig abgesaugt.

DNA-Markierung

Das Sediment jedes Röhrchens wird mit 100 Mikroliter folgender frisch angesetzter Lösung versetzt: 20 mM $MgCl_2$; 25 mM $NH_4Cl$; 25 mM Tris-HCl pH 7,6; 0,5 % Nonidet-P40; 0,2 % Beta-Mercaptoethanol; 2,5 mg/ml desoxy-Adenosintriphosphat; 2,5 mg/ml desoxy-Thymidintriphosphat; 2,5 mg/ml desoxy-Guanosintriphosphat;

...

10 Mikrocurie 125 Jod-desoxy-Cytidintriphosphat. Durch Auf- und Abziehen (mindestens zehnmal) der Lösung mit einer Pipette wird das Sediment gründlich suspendiert, danach werden die Sedimente vereinigt und in einem verschlossenen Plastikgefäß 3 Stunden bei 37° C inkubiert. Hierbei baut die HBV-DNA Polymerase radioaktives 125-Jod-desoxy-Cytidin in die HBV-DNA ein und füllt die Einzelstranglücke der HBV-DNA.

DNA-Freisetzung

Das Gemisch von 0,1 bis maximal 1 ml wird in einem 14 x 95 mm Ultrazentrifugenröhrchen auf einen diskontinuierlichen Saccharosegradienten, bestehend aus 6 ml 30 %iger Lösung, 5 ml 20 %iger und 1 bis 2 ml 10 %iger Lösung in 130 mM NaCl; 20 mM Tris-HCl; 0,1 % Rinderserumalbumin geschichtet. Nach 8 Stunden bei 38 000 U/Minute und 4° C wird der Überstand wieder sorgfältig abgezogen und zum Sediment 100 Mikroliter folgender frisch angesetzter Lösung gegeben: 40 mM Tris-HCl pH 7,5; 0,3 % Natriumlaurylsulfat; 40 mM EDTA; 2 mg/ml Proteinase K. Das Sediment wird wie vorher gründlich resuspendiert und dann 4 Stunden bei 37° C inkubiert, wobei die HBV-DNA freigesetzt wird.

Phenolextraktion

Die HBV-DNA wird nun durch Phenolextraktion gereinigt. Die gesamte Lösung, etwa 120 µl, wird in ein 1,5 ml-V-förmiges Zentrifugengefäß gegeben und dann wird dasselbe Volumen an Phenol, das mit Puffer verflüssigt ist, zugesetzt. Die beiden Flüssigkeiten werden durch Vibrieren oder Rotieren gründlich vermischt. Hierfür ist ein Vortex Mischer geeignet. Nun wird die Emulsion wieder getrennt, indem etwa 30 Sekunden in einer Eppendorfzentrifuge

...

zentrifugiert, das heißt, es wird voll beschleunigt bis etwa 2000 U/Minute und dann abgebremst. Die obere wässrige Phase wird abgenommen und in einem neuen Gefäß erneut mit Phenol extrahiert. Danach wird in der beschriebenen Weise zweimal mit Diethylether, der mit Wasser gesättigt war, extrahiert, wobei nun die wässrige Phase unten ist.

Alkoholfällung

Die verbliebene wässrige DNA-Lösung, etwa 100 µl, wird pro 100 µl mit 25 µl 3M Natriumacetat und mit 20 µl einer wässrigen transfer-RNA-Lösung mit 1 mg/ml als Trägersubstanz versetzt, danach mit 330 µl Ethanol. Das Gemisch wird auf -70 bis -80° C abgekühlt und nach 15 Minuten wieder in einem V-Gefäß bei Raumtemperatur 5 Minuten bei maximaler Drehzahl, zum Beispiel 12 000 U/Minute, abzentrifugiert. Der Überstand wird völlig abgesaugt, das Sediment wird im Vakuum kurz getrocknet und in 50 µl 10 mM Tris-HCl pH 7,4 1 mM EDTA aufgelöst und kühl gelagert. Wegen des radioaktiven Zerfalls soll innerhalb von einer Woche weitergearbeitet werden.

Restriktionsanalyse

Bevor die HBV-DNA kloniert werden kann, muß sie mit einem Restriktionsenzym geschnitten werden, das möglichst nur eine Schnittstelle in der HBV-DNA hat. In einem Probensatz werden 2 bis 3 µl der HBV-DNA mit 0,5 µg DNA des Bakteriophagen Lambda (im Handel erhältlich) in 2 µl $H_2O$ mit etwa 10 Einheiten BamHI und mit 5 µl Reaktionspuffer (20 mM Tris-HCl pH 8,0; 200 mM NaCl; 10 mM $MgCl_2$; 2 mM beta-Mercaptoethanol) versetzt, so daß etwa 10 µl Endvolumen erhalten werden. Ein Kontrollansatz ohne BamHI wird gleich behandelt. Nach 1 Stunde Inkubation bei

...

37° C werden 10 µl Elektrophoresepuffer mit 1 % Natriumlaurylsulfat, 10 % Glyzerin und 0,1 % eines Orange-Farbstoffes, der üblicherweise als Markerfarbstoff für elektrophoretische Wanderungen verwendet wird, zum Beispiel Orange G (Merck, Bestell-Nr. 6878) zugesetzt und die Mischung in eine Elektrophoreseauftragstasche gegeben. Parallel dazu sollte eine Probe mit einem Gemisch von DNA-Fragmenten bekannter Größe aufgetragen werden, zum Beispiel Lambda-DNA, die mit HindIII gespalten ist.

Die Elektrophorese erfolgt mit 1,1 % Agarosegel, das durch Erhitzen von 2,2 g Agarose mit 200 ml Elektrophoresepuffer hergestellt wurde. Der Puffer ist: 40 mM Tris-Base; 20 mM Natriumacetat; 1 mM EDTA; pH mit Eisessig auf 8,2 einstellen. Die Elektrophorese selbst kann in jeder Laborüblichen Horizontal- oder Vertikal-GelelektrophoreseApparatur vorzugsweise in Flachgelen durchgeführt werden, wobei die Hinweise des Herstellers zu beachten sind.

Die Wanderungsstrecke in der betreffenden Apparatur sollte zwischen 15 und 30 cm liegen, die Geldicke zwischen 2 und 6 mm, der Probenauftrag liegt auf der Minus-Pol-Seite und sollte mindestens 20 µl Probe fassen können. Man läßt bei rund 4 bis 6 Volt/cm Feldstärke laufen, bis nach einigen Stunden das Orange G als Zone das Ende des Gels in Richtung Plus-Pol erreicht hat. Dann wird das Gel entnommen und 30 Minuten in 1 Liter Ethidiumbromidlösung (500 µg/Liter) gebadet. Dabei färben sich die DNA-Banden der zur Probe beigefügten Lambda-DNA, die ebenfalls mit BamHI verdaut wurden, sowie die Banden der bekannten DNA-Fragmente in der Vergleichsspur. Durch Beleuchtung mit langwelligem UV-Licht werden die DNA-Fragmente als rote fluoreszierende Banden sichtbar. Die Menge der HBV-DNA reicht allerdings unter Umständen nicht aus, um hier sichtbar zu werden. Es ist vorteilhaft, das Bandenmuster mit

...

einem Orangefilter im Dunkeln bei Belichtungszeiten zwischen 10 und 240 Sekunden zu fotografieren. Zur Darstellung der HBV-DNA wird das Gel nach einer der üblichen Methoden getrocknet, wobei es auf einen stabilen Filterkarton oder eine hydrophile Kunststoffolie (zum Beispiel Gelbond) gelegt wird. Bei Verwendung von Filterkarton muß ein handelsüblicher Geltrockner mit Vakuumanschluß verwendet werden. Bei Verwendung von Gelbondfolie wird die Flüssigkeit durch Auflegen von Filterpapier herausgesaugt. Das getrocknete Gel bleibt als dünne Schicht auf dem Gelträger und wird nun im Dunkeln mit einem Röntgenfilm und einer Röntgenverstärkerfolie in engem Kontakt gebracht, zum Beispiel mit Kodak XAR-Film und Dupont Hiplus-Verstärkerfolie und einer Röntgenkassette. Relative Positionen des Films und des Gels zueinander werden durch einen Nadelstich markiert und durch Klebeband fixiert. Die mit 125 Jod markierte HBV-DNA schwärzt an den entsprechenden Stellen des Gels den Röntgenfilm. Der Film wird nach einigen Stunden bis Tagen entwickelt.

Die HBV-DNA ist ohne Restriktionsschnitt teils circulär, teils linear, so daß im Gel beziehungsweise am Röntgenfilm zwei Banden sichtbar werden: Eine, die einer DNA-Fragmentgröße von etwa 4000 Basenpaaren entspricht, die der circulären Form entspricht und eine zweite, die den etwa 3000 bis 3200 Basenpaaren der linearen Form entspricht, also etwas weiter gewandert ist. Nach einem Schnitt an einer Stelle wird die circuläre DNA in eine lineare DNA umgewandelt, so daß die Gelbande der circulären DNA im BamHI verschwindet.
Schneidet das Restriktionsenzym an mehr als einer Stelle, so entstehen neue DNA-Fragmente mit weniger als 3000 Basenpaaren. Das HBV-DNA-Isolat aus Blutkonserve F 991 wurde für die weitere Bearbeitung ausgewählt, weil es offensichtlich nur eine BamHI-Schnittstelle hatte. Nur

...

für solche Isolate ist das weitere Vorgehen geeignet. Die restliche HBV-DNA-Menge von etwa 45 µl wird in demselben Puffer wie vorher mit 100 Einheiten BamHI in 100 µl Endvolumen verdaut, jedoch wird keine Lamda-DNA zugefügt und das Volumen entsprechend vergrößert. Nach einer Stunde bei 37° C wird BamHI durch Phenol- und Ether-Extraktion wie vorher beschrieben entfernt. Diese BamHI geschnittene HBV-DNA wird für die Rekombination und Klonierung verwendet.

DNA-Rekombination

Als Vector wird das im Handel erhältliche Plasmid pBR322 verwendet. 6 µg reine Plasmid-DNA werden, wie im Absatz vorher beschrieben, mit 10 Einheiten BamHI in 50 µl geschnitten, durch Phenolextraktion und Ethanolpräzipitation gereinigt und in 50 µl $H_2O$ aufgenommen. Dazu werden 200 µl 100 mM Tris-HCl pH 8,0 und 2 µl bakterielle alkalische Phosphatase (15,7 mg/ml; 45 Einheiten/mg in Ammoniumsulfatlösung, Hersteller Worthington) zugesetzt. Die Phosphatase war vorher 25 Minuten auf 65° C erhitzt worden, um verunreinigende Desoxyribonucleasen zu inaktivieren. Das Reaktionsgemisch wurde 60 Minuten bei 65° C inkubiert und dann wie vorher mit Phenol und Ether extrahiert. 30 µl dieser desphosphorylierten Plasmid-DNA und 50 µl BamHI geschnittener HBV-DNA werden gemischt und zunächst mit Ethanol, wie vorher beschrieben, gefällt. Die getrocknete DNA wird mit 50 µl folgender frisch angesetzter Lösung aufgenommen: 30 mM Tris-HCl pH 7,6; 15 mM $MgCl_2$; 50 mM KCl; 20 mM Dithiothreitol; 20 µg/ml Rinderserumalbumin reinst; 1 mM Adenosintriphosphat, 0,2 Einheiten T 4 - DNA-Ligase (im Handel erhältlich). Die Mischung wird 24 Stunden bei 12,5° C inkubiert.

...

Transformation der E.coli-Zellen

Das benötigte LB-Medium besteht aus 1 % Bactotrypton (pankreatischer Verdau von Milchcasein); 0,5 % Hefe-Extrakt, 1 % NaCl in destilliertem $H_2O$ und ist 20 Minuten bei 120° C autoklaviert. Eine Kolonie von E.coli K12 Stamm 490 (oder einem anderen für diese Zwecke üblichen E.coli-Stamm) wird in 100 ml LB-Medium in einem 1-Liter-Erlenmeyerkolben übergeimpft und über Nacht unter Schütteln bei 37° C kultiviert, wobei der sterile Kolben mit lose sitzenden Aluminiumkappen abgedeckt ist. Von dieser Vorkultur werden 6 ml auf 400 ml LB-Medium in einen 2 Liter Erlenmeyerkolben übertragen. Dieser wird bei 37° C geschüttelt, wobei alle 20 Minuten die optische Dichte (OD) bei 550 nm gemessen wird. Bei Erreichen einer OD 550 von 0,6 bis 0,7 wird der Kolben durch Schwenken in Eiswasser auf 0° C abgekühlt. Dann werden die Bakterien in der Kälte 10 Minuten bei 6000 U/Minute abzentrifugiert. Die Zellen werden in einem kleinen Volumen eiskalter 15 mM NaCl-Lösung suspendiert und dann wird mit eiskalter 15 mM NaCl auf 400 ml aufgefüllt. Die Zellen werden erneut wie oben bei 4° C abzentrifugiert und nun in 200 ml eiskalter 60 mM $CaCl_2$-Lösung aufgenommen und 20 Minuten auf Eis belassen. Dann wird wieder abzentrifugiert und in 40 ml eiskalter 60 mM $CaCl_2$-Lösung suspendiert. Die Zellen können bis zur Verwendung maximal bis 1 Tag auf Eis aufbewahrt werden. 200 µl dieser Zellen werden in einem sterilen Plastik-Reagenzröhrchen mit 15 bis 30 µl der rekombinierten DNA, wie sie nach der Ligase-Reaktion vorliegt, vermischt und 30 Minuten auf Eis belassen. Danach wird das Röhrchen exakt 120 Sekunden in ein 42° C warmes Wasserbad gebracht und dann wieder 5 Minuten auf Eis gestellt. Dann werden 2 ml LB-Medium zugegeben und der Ansatz 1 Stunde bei 37° C inkubiert. Nun werden 100 µl davon auf 8 cm

...

große Petrischalen, die 10 ml Nährboden enthalten, ausgestrichen. Der halbfeste Nährboden besteht aus 1,5 g Agar, 10 g Bactotrypton, 10 g Hefeextrakt, 5 g NaCl; 1 Liter destilliertem $H_2O$. Die Komponenten werden gemischt, 20 Minuten bei 120° C autoklaviert und auf 55° C abgekühlt. Dazu werden 4 ml einer sterilen Lösung von 25 mg/ml Ampicillin (Natriumsalz) in Wasser zugemischt. 10 ml dieser Lösung werden in sterile Petrischalen ausgegossen und erstarren lassen. Die Platten halten sich bei 4° C 2 Wochen.

Die bestrichenen Platten werden über Nacht bei 37° C in den Brutschrank gelegt. Aus den erschienenen Kolonien müssen nun jene herausgefunden werden, die tatsächlich Plasmide in Form von rekombinierter DNA haben. Durch BamHI wird das Resistenzgen gegen Tetracyclin im Plasmid pBR 322 zerstört. Wenn hier eine fremde DNA eingesetzt ist, verliert das Plasmid seine Tetracyclin-Resistenz. Es werden die erhaltenen Kolonien parallel in definierter Anordnung auf LB-Agar mit 20 µg Tetracyclin/ml geimpft, (1 Liter Nährboden von 55° C wird mit 2 ml einer Lösung von 10 mg Tetracyclin-HCl in 50 % Ethanol versetzt und in Petrischalen gegossen) und daneben noch einmal in der gleichen Anordnung auf LB-Ampicillin-Platten. Hierfür eignen sich pro einzelner Kolonie jeweils sterile Zahnstocher.

Nach Bebrütung über Nacht bei 37° C wird das Kolonie-Wachstum auf beiden Nährböden verglichen. Kolonien, die auf dem ampicillinhaltigen Nährboden, nicht aber auf den tetracyclinhaltigen gewachsen sind, sind Kandidaten für E.coli-Klone, die das gewünschte Plasmid mit eingebauter HBV-DNA enthalten.

Die endgültige Identifizierung erfolgt durch Isolierung und Restriktionsanalyse der Plasmide aus den Kandidatenklonen. Hierfür werden die Klone mit sterilen Zahnstochern

...

jeweils auf einem Sechstel der Fläche einer 8 cm Petrischale mit LB-Ampicillin-Agar verstrichen, über Nacht
bei 37° C kultiviert und mit sterilen Zahnstochern
abgeerntet und in einem V-Zentrifugengefäß mit 200 µl
Puffer (25 % Saccharose, 50 mM Tris-HCl pH 8,0) gründlich suspendiert. Dazu werden jeweils 66 µl Lysozymlösung (10 mg/ml) zugemischt. Nach 5 Minuten bei Raumtemperatur werden 54 µl 250 mM EDTA pH 8,0 zugemischt.
Nach 5 Minuten wird 320 µl Detergenslösung zugefügt:
0,1 % Alkylphenylpolyethylenglykol (Triton X-100) als
Detergens; 7 mM EDTA; 50 mM Tris-HCl pH 8,0.
Nach 15 Minuten bei 65° C im Wasserbad werden die
Lösungen bei 4° C 20 Minuten lang bei maximaler Drehzahl
zum Beispiel 12 000 U/Minute geklärt. Die Überstände
werden in neue V-Gefäße überführt und mit einem gleichen
Volumen Isopropanol (etwa 550 µl) vermischt, 2 Stunden
bei -20° C stehen gelassen und das DNA-Präzipitat wird
5 Minuten bei Maximaldrehzahl abgeschleudert. Der Überstand wird vollständig abgenommen, im Vakuum kurz getrocknet und in 75 µl $H_2O$ wieder aufgelöst. Dieses
sogenannte Minilysat liefert Plasmid-DNA, die für die
Restriktionsanalyse geeignet ist.

10 µl des Minilysats werden mit den vorher beschriebenen
Bedingungen mit BamHI verdaut und elektrophoretisch in
1,1 % Agarosegel wie vorher beschrieben analysiert. Die
DNA-Fragmente des Plasmids werden durch Färbung mit
Ethidiumbromid sichtbar gemacht. Als Vergleichssubstanz
wird 1 µg HindIII gespaltene Lambda-DNA (im Handel
erhältlich) mit analysiert. Wenn eine Kolonie ein Plasmid
enthält, das die volle 991-DNA oder eine HBV-DNA mit
einer BamHI-Stelle eingebaut hat, entsteht neben dem
4000 Basenpaar-Fragment von PBR322 ein 3200 BP-Fragment
im entsprechenden Minilysat. Es ist klar, daß nicht
alle, vermutlich sogar nur wenige HBV-DNA-Isolate, nur
eine BamHI-Stelle haben. Um exakt den gleichen experimenteller

...

Weg beschreiten zu können, müssen relativ viele HBV-Isolate untersucht werden. Alternativ können analoge Experimente mit EcoRI und anderen Plasmiden, zum Beispiel pACYC 184, durchgeführt werden.

Ein ausgewählter E.coli-Stamm mit dem Plasmid pHBV 991 wird auf LB-Ampicillin Agar ausgestrichen und zur Erhaltung der Lebensfähigkeit alle 3 Monate auf neue Nährboden überimpft. Die Kulturen müssen vor dem Austrocknen gesichert sein. Eine Lagerung bis zu 1 Jahr bei -20° C ist möglich, wenn eine LB-Ampicillin Flüssigkultur mit 20 % Glycerin versetzt wird.

Präparation der pHBV 991 DNA

Wie vorher beschrieben, wird in 100 ml LB-Ampicillin-Medium eine Übernachtkultur von 200 ml des E.coli-Stamm mit pHBV 991 angelegt. Mit 20 ml dieser Vorkultur werden 400 ml LB-Ampicillin-Medium in einem 2 Liter-Kolben beimpft, parallel können bis zu 6 Erlenmeyerkolben mit 400 ml verwendet werden. Die weiteren Angaben gelten pro Kolben. Die Vermehrung erfolgt unter Schütteln bei 37° C. Die OD600 wird verfolgt, am bequemsten in einem parallel mitgefärbten Klettkolben, der einen seitlichen Ansatz hat, der direkt in ein Photometer gebracht werden kann. Bei einer OD600 von 0,4 (nach 2 bis 3 Stunden) werden pro Kolben 2,5 ml Chloramphenicol (34 mg/ml in Ethanol) zugefügt, dann wird über Nacht bei 37° C weitergeschüttelt. Dann werden die Zellen bei 6000 U/Minute für 10 Minuten bei 4° C abzentrifugiert. Der Überstand wird abgegossen, die Zellen werden in insgesamt 100 ml eiskaltem Puffer (130 mM NaCl; 10 mM Tris-HCl, pH 7,4; 1 mM EDTA) suspendiert und wieder abzentrifugiert. Der Überstand wird weggegossen und das Pellet mit 9 ml Lyse-Puffer (50 mM Glucose; 25 mM Tris-HCl pH 8,0; 10 mM EDTA)

...

suspendiert und dann wird 1 ml Lysozymlösung (50 mg in 1 ml Lyse-Puffer) zugegeben. Nach 5 Minuten bei Raumtemperatur werden 20 ml Denaturierungslösung (1 % Natriumlaurylsulfat; 200 mM NaOH) zugefügt, 10 Minuten auf Eis unter leichtem Schwenken gelagert. Dann werden 15 ml hochmolare Acetatlösung (3 M Kaliumacetat, 2 M Essigsäure) zugefügt, kräftig gerührt und weitere 10 Minuten auf Eis gehalten. Dann wird 20 Minuten bei 20 000 U/ Minute und 4° C zentrifugiert. Der Überstand ist "klares Lysat", das mit 0,6 Volumen Isopropanol vermischt wird. Nach 15 Minuten bei 20 bis 25° C wird das Präzipitat bei 12 000 U/Minute bei 20° C 20 Minuten zentrifugiert, der Überstand verworfen, das Sediment mit 70 %igem Ethanol gewaschen, im Vakuum kurz getrocknet und in 8 ml 10 mM Tris 1 mM EDTA (TE) aufgelöst.

Pro jedem ml dieser DNA-Lösung werden 1 g festes Cäsiumchlorid hinzugefügt und gelöst und jedem ml dieser CsCl-Lösung werden 80 µl Ethidiumbromidlösung (10 mg/ml) zugegeben. Die Dichte soll 1,55 g/$cm^3$ betragen und wird notfalls mit TE-Puffer oder CsCl nachgestellt. Diese Lösung wird in durchsichtige verschließbare Zentrifugenröhrchen (zum Beispiel Quickseal 16 x 76 mm von Beckmann) gebracht, notfalls werden die Röhrchen mit Paraffinöl randvoll gemacht. Nach 36 Stunden bei 20° C und 45 000 U/Minute in einem Winkelrotor (zum Beispiel Beckmann Ti 70.1) werden die Röhrchen entnommen, wobei meist schon bei Normallicht zwei rote Zonen sichtbar werden. Die untere Zone wird durch Anstechen mit einer Injektionsnadel abgenommen, wobei durch Einstechen einer zweiten Nadel von oben Luft zugeführt wird. Das Ethidiumbromid wird aus der so gewonnenen Lösung durch 5 maliges Ausschütteln mit dem gleichen Volumen wassergesättigtes n-Butanol extrahiert; das Cäsiumchlorid wird durch je 2 stündige Dialyse in einer Kollodiumhülse (Sartorius)

...

gegen 3 mal 100 ml TE-Puffer entfernt. Die pHBV 991 DNA wird dann mit Ethanol gefällt und in 50 µl TE-Puffer pro 400 ml Ausgangskultur aufgelöst. Die DNA-Konzentration dieser Lösung wird in einer geeigneten Verdünnung UVphotometrisch bestimmt, wobei eine OD280 von 1 50 µg reiner DNA/ml entsprechen.

Bei dem Klon K490 pHBV991 besteht die Prä-S(1)-Nucleotidsequenz aus 119 Codons.
Der Klon ist unter der Bezeichnung K490 pHBV991 bei der Sammelstelle "Deutsche Sammlung von Mikroorganismen (DSM), Griesebachstraße 8, 3400 Göttingen, Bundesrepublik Deutschland" hinterlegt und hat die Hinterlegungsnummer DSM 3082.

Anstelle des hier verwendeten Stammes 490 von E.coli K12 können auch alle anderen für die Gelklonierung geeigneten E.coliK-Abkömmlinge verwendet werden.

...

2. <u>Herstellung der Prä-S(1)-Fragmente aus pHBV991</u>

a) BstEII/Bal31/EcoRI-Fragment
260 µg Hepatitis B-DNA (Klon pHBV991) in 200 µl, 10 mM Tris-HCl pH 7,4 und 1 mM EDTA (Puffermischung TE) wurden mit 600 Einheiten BstEII unter Zugabe von 200 µl Reaktionspuffer 5 Stunden bei 60° C verdaut. Der Reaktionspuffer enthält 200 mM Tris-HCl pH 7,4; 100 mM KCl; 10 mM $MgCl_2$. Die Lösung wurde dann auf eine kleine Chromatographiesäule mit etwa 1 ml Diethylaminoethyl-Cellulose in TE-Puffer gegeben. Nachdem die Lösung eingezogen war, wurde mit 5 ml TE gewaschen. Danach wurden dreimal 0,5 ml 2 M NaCl in TE nacheinander aufgegeben und das Eluat getrennt aufgefangen. Daraus wurde durch Zugabe von jeweils 1 ml Ethanol die DNA wie früher, jedoch ohne weiteren Salzzusatz, gefällt und wieder in insgesamt 275 µl TE aufgelöst. Nun wurden 275 µl Puffer aus 24 mM $CaCl_2$; 24 mM $MgCl_2$; 400 mM NaCl; 40 mM Tris-HCl, pH 8,0, 2 mM EDTA; 0,5 mg/ml Rinderserumalbumin zugesetzt und das Gemisch auf 30° C vorgewärmt. Nach Zugabe von genau 0,6 Einheiten Bal31 wird genau 16 Minuten und bei einem zweiten ansonsten identischen Ansatz 18 Minuten verdaut. Da die Bal31 linear mit der Zeit die Enden verkürzt, sollte durch die zwei verschiedenen Zeiten die Wahrscheinlichkeit erhöht werden, daß Fragmente der richtigen Länge erhalten werden. Jede einzelne Bal31-Charge muß erst in Probeansätzen auf ihre Wirkung geprüft werden. Es sollten rund 28 Basen pro Ende entfernt werden.

...

Die Reaktionen wurden jeweils durch Zugabe von 70 µl
250 mM Na$_2$EDTA gestoppt, die Mischungen der beiden
Ansätze dann 10 Minuten auf 60° C erhitzt und dann
2 Stunden in einer Kollodiumhülse gegen 100 ml TE
dialysiert. Danach wurde die DNA mit EcoRI verdaut.
Es wurde das gleiche Volumen Puffer mit 200 mM
Tris-HCl pH 7,5; 100 mM NaCl; 20 mM MgCl$_2$ und
1000 Einheiten EcoRI zugegeben und für 2 Stunden
bei 37° C inkubiert.

EcoRI wurde durch Erhitzen (10 Minuten/60° C) inaktiviert. Die DNA wurde mit dem doppelten Volumen
Ethanol präzipitiert, in 20 µl 6,6 mM Tris-HCl
pH 7,4, 6,6 mM MgCl$_2$, 1 mM Dithiothreitol, 5 mM
NaCl, 100 µM Desoxyadenosintriphosphat, 100 µM
Desoxythymidintriphosphat aufgelöst und mit
0,2 Einheiten Klenow-Polymerase 16 Stunden
bei 14° C inkubiert. Die etwa 370 bis 400 Basenpaare großen DNA-Fragmente wurden in 5 % Polyacrylamidgel elektrophoretisch aufgetrennt. Gel- und Kammerpuffer
waren 60 mM Tris-Borat, 60 mM Borsäure, 1 mM
EDTA, pH 8,3 (TBE). Das Gel war 20 cm lang,
18 cm breit und 1 mm dick. Die Probenauftragstaschen waren 8 mm breit und wurden mit jeweils
10 µl beschickt. Die Proben wurden vor der Elektrophorese mit 0,2 Volumen von 50 % Glycerin; 0,05 %
Bromphenolblau in TBE versetzt. Die Elektrophorese
erfolgte bei 240 Volt bis nach 2 bis 4 Stunden das
Bromphenolblau die Anodenseite des Gels erreicht
hat. Danach befinden sich die gewünschten Fragmente
im mittleren Drittel, wo sie durch Färbung mit
Ethidiumbromidlösung (0,5 µg/ml) im UV-Licht sichtbar gemacht wurden. Die Fragmentzonen wurden ausgeschnitten, zerkleinert und gewogen, dann mit
demselben Volumen 0,5 M Ammonacetat; 1 mM EDTA

...

versetzt und über Nacht bei 37° C über Kopf rotiert. Danach wurde scharf bei 10 000 U/Minute 10 Minuten bei 20° C abzentrifugiert. Der Überstand wurde mit 2 Volumina Ethanol präzipitiert und in 20 µl TE gelöst.

b) BstEII/EcoRI Fragment

50 µg pHBV 991 DNA in 40 µl TE wurden mit 40 µl 200 mM Tris-HCl pH 7,4; 100 mM KCl; 10 mM $MgCl_2$ und mit 120 Einheiten BstEII versetzt und 2 Stunden bei 60° C im Wasserbad inkubiert, dann wurden 20 µl 250 mM NaCl, 50 mM $MgCl_2$ und 200 Einheiten EcoRI zugesetzt und weitere 2 Stunden bei 37° C verdaut. Dann wird die Lösung 5 Minuten auf 70° C im Wasserbad erhitzt, danach wird in einer Mikrokollodiumhülse 1 Stunde gegen 100 ml TE dialysiert. Diese Lösung (etwa 150 µl) wurde mit dem gleichen Volumen 60 mMol Natriumacetatpuffer pH 4,46; 100 mM NaCl; 2 mM $ZnCl_2$; 10 % Glycerin-Puffer und mit 100 Einheiten Mung Bohnen Nuclease versetzt und 10 Minuten bei 37° C inkubiert. Danach wurden 30 µl 250 mM $Na_2EDTA$ zugefügt, das Gemisch wie unter 1) angegeben ist (siehe Phenolextraktion, Alkoholfällung) mit Phenol/Ether extrahiert und mit Ethanol präzipitiert. Die DNA wurde in 20 µl TE aufgenommen und das 400 Basenpaare lange Fragment wie unter 2a) elektrophoretisch isoliert und nach Ethanolpräzipitation in 20 µl TE aufgelöst.

c) Alu I/EcoRI Fragment

Der folgende Schritt dient der Vermehrung des beziehungsweise der DNA-Fragmente, die die Prä-S(1)-Sequenz enthalten (die zum Beispiel gemäß 2b) oder auch 2a) erhalten wurden), wobei außerdem aus solchen Fragmenten durch das Restriktionsenzym AluI überflüssige Basenpaare abgespalten werden.

...

Als Vector wurde das Plasmid pGL101 verwendet, das von Thummel et al beschrieben wurde (J.Virol. 37, 1981, 683-697). Das Plasmid wurde in E.coli JM101 wie unter 1) angegeben vermehrt, extrahiert und gereinigt. 10 µg pGL101 DNA in 15 µl TE wurden mit 15 µl Reaktionspuffer 12 mM Tris-HCl pH 7,4; 120 mM NaCl; 12 mM $MgCl_2$; 15 mM beta-Mercaptoethanol; 0,02 % Triton X100 und mit 20 Einheiten PvuII versetzt und 2 Stunden bei 37° C inkubiert. Das Gemisch wurde 10 Minuten auf 65° C erwärmt, um PvuII zu inaktivieren.

1 µg Fragment in 10 µl TE von Abschnitt 2b) wurde mit 0,3 µg DNA des Plasmids pGL101, die mit EcoRI-PvuII geschnitten war, vermischt und in 20 µl 50 mM Tris HCl pH 7,4, 10 mM $MgCl_2$, 1 mM Adenosintriphosphat mit 0,1 Einheiten T4-DNA Ligase 18 Stunden bei 4° C ligiert. Mit einem Drittel des Ansatzes wurde nach der Methode von Hanahan, E.coli Stamm JM101 (Amersham Braunschweig) transformiert und auf ampicillinhaltigen Platten selektioniert. Transformanten mit dem Prä-S(1)-Fragment wurden durch in situ Hybridisierung der Kolonien auf Nitrocellulosemembranen mit $^{32}$P-markierter Hepatitis B-Virus-DNA identifiziert. Von positiven Klonen wurden Mini-Lysate hergestellt und die darin enthaltene Plasmid-DNA mit AluI verdaut. Hierbei entsteht wieder ein 450 Basenpaar-Fragment, das im wesentlichen die Prä-S(1)-Sequenz enthält. Von einem ausgewählten Klon mit dem erwarteten 450 Basenpaar-Fragment im AluI-Verdau wurde gereinigte Plasmid-DNA in präparativem Maßstab hergestellt, 50 µg davon mit AluI verdaut und das gewünschte DNA-Fragment mit der Prä-S(1)-Sequenz durch Gelelektrophorese isoliert und nach Ethanolpräzipitation in 20 µl TE gelöst.

...

3. **Expression des Prä-S(1)-Polypeptids**

a) Transcriptionskontrolle durch den LacUV5 Promoter. Das BstEII/Bal31/EcoRI Fragment beziehungsweise das Alu I/EcoRI Fragment wurden wie unter Abschnitt 2c mit pGL101 ligiert und in E.coli JM 101 kloniert. Die richtige Polarität der Fragmentinsertion wurde durch Doppelverdau der jeweiligen Plasmid DNA mit EcoRI und Sau96I und Bestimmung der Fragmentgrößen ermittelt. Bei richtiger Polarität entsteht neben den Fragmenten mit 1400, 616, 222, 79, 18, 17 Basenpaaren ein 200 Basenpaar-Fragment, bei falscher Polarität stattdessen ein Fragment mit 380 Basenpaaren.

b) Transcriptionskontrolle durch den tac-Promoter. 50 µg Plasmid DNA pDR540 (Pharmacia-PL Biochemicals) wurden mit BamHI (New England Biolabs) verdaut, und die überstehenden Enden wie vorher beschrieben mit Mung-Bean-Nuclease verkürzt. 0,3 µg der mit Phenol extrahierten und Äthanol gefällten DNA wurden mit 1 µg des AluI/EcoRI Fragments wie vorher beschrieben ligiert und die rekombinierte DNA kloniert. Klone mit der richtigen Polarität wurden durch Verdau der Plasmide mit HindIII und BalI bestimmt. Bei richtiger Polarität wird ein 250 Basenpaar Fragment erhalten, bei falscher stattdessen ein 360 Basenpaar-Fragment.

c) Identifizierung der Prä-S(1)-Polypeptid produzierenden Klone. Klone mit der richtigen Polarität wurden auf Nitrocellulosemembranen ausgestrichen, die

...

Membranen auf Agar-Nährboden gelegt, der aus LB-Medium, 100 µg/ml Ampicillin und 1 mM Isopropylthioglactosid bestand, und über Nacht bei 37° C kultiviert. Die Kolonien wurden in situ durch Auflegen auf feuchtes Filterpapier mit 5 mg/ml Lysozym und Chloroformdampf in 15 Minuten bei Raumtemperatur lysiert. Die Membran wurde dann mit 1:4 verdünntem Hybridom-kulturüberstand des monoklonalen Antikörpers MA18/7 1 Stunde bei 37° C geschüttelt, 3mal mit 0,2 % TWEEN 20 gewaschen, mit Antikörper gegen Maus IgG, peroxidasemarkiert, (Dakopatts) 1:1000 in 20 % Kalbserum 1 Stunde bei 37° C geschüttelt, 3mal mit 0,5 % Detergens TWEEN 20 in PBS gewaschen und mit 0,01 % Diaminobenzidin, 0,03 % $H_2O_2$ gefärbt.
Die positiven Kolonien färbten sich deutlich stärker als negative Kontrollkolonien.

...

## Extraktion und Anreicherung des Prä-s(1)-Polypeptids

a) Für die Anreicherung des gewünschten Produkts wurde ein Immunabsorbens hergestellt. 3 g poröse Aminopropyl-Glasperlen (Glasperlen, die Aminopropylgruppen enthalten) mit 140 nm Porenweite (Fluka) wurden mit 6 % Glutardialdehyd in 0,3 M $NaHCO_3$ bei Raumtemperatur 30 Minuten geschüttelt, gründlich mit Wasser gewaschen und dann mit 50 mg des monoklonalen Antikörpers MA18/7 (mit den konstanten Merkmalen Immunoglobulin $G_1$/Kappa) aus Maus Ascites (gereinigt durch zweimalige Fällung mit 17 % Natriumsulfat) in 0,01 M $NaHCO_3$ pH 9,1 vier Stunden bei Raumtemperatur geschüttelt. Die Perlen wurden mit 0,13 M NaCl und 0,02 M Na-Phosphat pH 7,4 (Lösung PBS) gewaschen und dann mit 5 ml 0,1 % Natriumborhydrid in 0,01 M $NaHCO_3$ für 2 Stunden auf Eis gerührt. Dann wurden die Glasperlen in einer Minisäule zuerst mit 20 ml PBS, dann mit 2 ml 3 M NaSCN und anschließend nochmals mit 20 ml PBS gewaschen.

b) Ein Prä-S(1) exprimierender E.coli Klon, zum Beispiel pKG1 gemäß 3a) oder pKG2 gemäß 3b) wurde über Nacht in LB-Medium mit 100 µg/ml Ampicillin kultiviert und 10 ml davon auf 400 LB-Medium mit 1 mM IPTG in einem 2 Liter Kolben überimpft und bei 37° C geschüttelt. Bei einer optischen Dichte bei 600 nm von 2,5 wurden die Zellen abzentrifugiert und das Pellet im gleichen Volumen 10 mM Tris HCl pH 7,4, 1 mM EDTA (Pufferlösung TE) suspendiert, mit 5 mg/ml Lysozym und mit 1 % Nonidet P40 in TE versetzt. Nach 10 Minuten auf Eis wurde bei 12000 U/Minute 15 Minuten bei 4° C zentrifugiert. Der Überstand ist klares Lysat.

...

0180012

c) 6,5 ml klares Lysat wurden im Verlauf von einer Stunde durch 1 ml Immunadsorbens (gemäß a) hergestellt) in eine Minisäule (Maße 5 x 50 mm) gepumpt. Die Säule wurde nacheinander mit 2 ml TE, 2 ml 0,5 M NaCl, 20 ml $H_2O$ jeweils mit 0,2 % TWEEN 20 gewaschen. Dann wurde das Prä-S(1)-Polypeptid mit 2 ml 3 M NaSCN in TE-Puffer abgelöst, wobei 100 µl Fraktionen aufgefangen wurden. Die ersten drei Fraktionen enthalten etwa 10-fach angereichertes Prä-S(1)-Polypeptid in gereinigter Form. Das Eluat wird vereinigt, 2 Stunden gegen PBS-Lösung (0,13 M NaCl + 0,02 M Na-Phosphat pH 7,4) dialysiert und eingefroren.

Das erhaltene Polypeptid hat die Formel I gemäß Anspruch 8, worin X = MetGlnTrpLeuProArgAlaPheArg-OH ist.

...

Erläuterung von technischen Ausdrücken und Abkürzungen:

| | |
|---|---|
| Tris: | Trishydroxymethylaminoethan |
| EDTA: | Ethylendiaminotetraessigsäure |
| TE: | wässrige Puffermischung, die pro Liter 10 mMol Tris-HCl pH 7,4 und 1 mMol EDTA enthält |
| LB-Medium: | Dieses Medium besteht aus 1 % eines pankreatischen Verdaus von Milchcasein (Bactotrypton), 0,5 % Hefeextrakt und 1 % NaCl in destilliertem Wasser und wurde 20 Minuten im Autoklaven auf 120° C erhitzt |
| PBS: | Wässrige Puffermischung, die pro Liter 130 mMol NaCl und 20 mMol Natriumphosphat pH 7,4 enthält. |
| TWEEN 20: | Polyoxyethylen(20)-sorbitanmonolaurat |
| Offener Ableserahmen: | Längste mögliche kontinuierliche Abfolge von Codons, die mit einem Startcodon beginnt und mit einem Stopcodon endet. |
| Codon: | Dreiereinheit von Nucleotidbasen, die eine Aminosäure in einer Polypeptidsequenz codieren oder als eines der drei Stopcodons (TAA, TGA, TAG) den Abbruch der Proteinbiosynthese codieren. |
| Startcodon (ATG): | Erforderlich für den Beginn der Proteinbiosynthese, codiert auch die Aminosäure Methionin am Aminoende der Proteinsequenz oder innerhalb der Proteinsequenz. |

...

| | |
|---|---|
| EcoRI: | Restriktionsendonuclease (zum Beispiel Firma BRL) |
| BstEII: | Restriktionsendonuclease (zum Beispiel Firma BRL) |
| BGlII: | Restriktionsendonuclease (zum Beispiel Firma BRL) |
| Bal31: | Doppelstrangspezifische Exonuclease (Firma BRL) |
| Klenow-Polymerase: | DNA-Polymerase Fragment nach Klenow (Firma Boehringer-Mannheim) |
| Mung-Bean-Nuclease: | Einzelstrangspezifische Exonuclease (Firma PL-Biochemicals) |
| AluI: | Restriktionsenzym (Firma BRL) |
| T4-DNA-Ligase: | Enzym, das verschiedene DNA-Moleküle verbindet (Firma Biolabs) |
| Sau 96I: | Restriktionsendonuclease (Firma New England Biolabs) |
| BamHI: | Restriktionsendonuclease (Firma New England Biolabs) |
| HindIII: | Restriktionsendonuclease (Firma New England Biolabs) |
| BalI: | Restriktionsendonuclease (Firma New England Biolabs) |
| Expressionsplasmid pGL101: | Gegenüber seinem Ausgangsplasmid pBR322 ist es durch einen EcoRI-PvuII-Restriktions-Schnitt um 2066 Basen verkürzt. In die entstandene Lücke wurde unter Regenerierung der EcoRI- und PvuII-Stelle ein DNA-Fragment insertiert, das den induzierbaren Lac UV5-Promoter trägt. Diese Variante des in E.coli natürlich vorkommenden Lac-Promoters kann Gene unabhängig von der Bindung des CAP-Genproduktes exprimieren. Literaturstelle: J.Virol. 37 (1981), 683-697. |

...

Beispiel II

Herstellung eines Polypeptids, welches die Prä-S(1)-
und die Prä-S(2)-Aminosäuresequenz enthält aus Hepatitis B-
Virus (HBV) oder Hepatitis B-Virus-Oberflächenantigen (HBsAg).

Das Peptid gemäß diesem Beispiel ist das Prä-S-Polypeptid, welches aus der Prä-S(1)- und der Prä-S(2)-
Aminosäuresequenz gemäß Fig. 1 besteht plus den beiden
Aminosäuren Methionin und Glutaminsäure im Anschluß
an Prä-S(2).

Geeignet sind als Ausgangsmaterial besonders HBV-
Partikel oder HBsAg-Filamente, die in üblicher Weise
oder wie am Schluß dieses Beispiels angegeben ist
gereinigt wurden. Geeignet sind auch gereinigte HBsAg
20 nm Partikel, soweit diese Partikel ausreichende
Mengen der Proteine P39 und GP42 enthalten. Der Proteingehalt der jeweiligen Probe wird durch UV-Photometrie
bei 280 nm Wellenlänge festgestellt, wobei ein
Extinktionskoeffizient von 4,3 für eine Lösung von
1 mg/ml HBV- oder HBsAg-Protein angenommen wurde.
40 Mikrogramm gereinigte HBV-Partikel oder 400 µg
gereinigte HBsAg Filamente oder 4 mg HBsAg 20 nm
Partikel in 0,4 ml wässriger Lösung, die 130 mM NaCl,
20 mM Natriumphosphat pH 7,5 enthält (Pufferlösung PBS)
wurde mit 0,1 ml einer wässrigen Lösung von 500 mM
Tris-HCl pH 7,4; 0,5 % Natriumdodecylsulfat; 5 %
Dithiothreit und danach mit 0,125 mg reiner Protease
aus Staphylococcus aureus V8 in 50 µl PBS versetzt.
Das Gemisch wurde bei 37° C inkubiert. Nach 24 Stunden
und nach 48 Stunden wurden jeweils weitere 50 µl Proteaselösung zugesetzt. Nach 72 Stunden wurde die Lösung mit
0,6 ml einer wässrigen Lösung von 6 % Natriumdodecylsulfat,
10 % Dithiothreit, 140 mM Tris-HCl pH 6,8; 22 %

...

Glycerin; 0,03 % Bromphenolblau versetzt und 5 Minuten auf 95° C erhitzt. Danach wurde die Lösung in Aliquoten von 50 μl einer Elektrophorese durch 15 %iges Polyacrylamidgel unterzogen. Das Trenngel hatte 1,5 mm Dicke, war 18 cm breit und 24 cm lang. Es hatte am Minuspol 10 Probetaschen. Eine außen liegende Probentasche enthielt ein Proteinmarkergemisch mit bekannten Molgewichten. Das Puffersystem ist von Laemmli beschrieben (Nature Band 227 (1970), Seiten 680 - 685). Die Elektrophorese erfolgte bei 3 - 6 Volt/cm über Nacht. Dann wurde das Gel entnommen, ein 2 cm breiter Längsstreifen mit einem aufgetrennten Aliquot und den Proteinmarkern ausgeschnitten und mit Silber nach der Methode von Merill et al. (Science 211 (1981) 1437) gefärbt. Durch Vergleich mit den Proteinmarkern bekannten Molgewichts ließ sich die 18 000 Dalton Bande des Prä-S-Fragments im Protease Verdau identifizieren. Die entsprechende Zone im nicht angefärbten Gelanteil wurde ausgeschnitten, mit einem Zellhomogenisator mit rotierendem Pistill (nach Potter-Elvehjem) zerkleinert und mit 3 Volumina 0,1 M Ammoniumbicarbonatlösung in Wasser 2 Stunden geschüttelt. Nach scharfem Abzentrifugieren (10 000 U/min) wurde der Überstand mit dem Prä-S Proteinfragment gefriergetrocknet.

Da das Prä-S Polypeptid mit 18 000 Dalton nur aus den Proteinen P39 und GP42, nicht aber aus den anderen Proteinen des HBV oder HBsAg hervorgeht, muß gesichert sein, daß das Ausgangsmaterial diese Proteine enthält. Dies läßt sich am geeignetsten durch analytische Polyacrylamidgelelektrophorese mit 0,75 mm dicken Gelschichten, ansonsten wie vorher beschrieben, feststellen. Wenn man 2 μg reines HBsAg-Protein pro Elektrophoresetasche startet, muß in der Silberfärbung bei den Positionen von etwa 39 000 und 42 000 Dalton eine

...

deutlich erkennbare gefärbte Bande erkennbar werden.

Die Reinigung der HBV-Partikel und der HBs-Filamente erfolgte auf folgende Weise:

Menschliches Blutplasma (etwa 150 ml) mit mehr als $10^8$ Hepatitis B-Viren pro ml wurde mit TNE-Puffer (0,13 M NaCl, 10 mM Trishydroxyaminomethan HCl, pH 7,4, 1 mM EDTA in Wasser) durch eine Chromatographiesäule (10 cm dick, 100 cm lang) mit perlförmigem 6 %igen Agarosegel (zum Beispiel Biogel A5M) gepumpt. Die aufgefangenen 500 Fraktionen von 15 ml wurden auf virales Core Antigen mit dem Enzymimmuntest (J.Virol. Band 42, 1982, Seite 761) untersucht, wobei die Virushülle vor dem Test durch Behandlung mit 0,5 % Nonidet P40 und 0,3 % ß-Mercaptoethanol (jeweils Endkonzentration) vorher abgespalten wurde. Die positiven Fraktionen wurden vereinigt und je 30 ml davon in 10 bis 12 Zentrifugenröhrchen mit 38 ml Fassungsvermögen auf 8 ml 30 %ige Saccharoselösung in TNE-Puffer (Gewicht/Gewicht) geschichtet. Die Röhrchen wurden im Schwingbecherrotor (zum Beispiel SW28 der Firma Beckmann) 24 Stunden bei 25 000 U/min und 10° C zentrifugiert. Der Überstand wurde vollständig entfernt, das Sediment von je 6 Röhrchen in 1 ml TNE-Puffer suspendiert. Diese Virussuspension wurde auf einen Saccharose-Konzentrationsgradienten von 30 bis 55 % in TNE-Puffer in einem 13 ml Zentrifugenröhrchen (zum Beispiel für den Rotor SW 42 Ti der Firma Beckmann) geschichtet und 20 Stunden bei 34 000 U/min und 10° C zentrifugiert. Es wurden Fraktionen von 400 µl nach Anstechen des Röhrchenbodens gesammelt, 25 µl Anteile der Fraktionen wurden quantitativ auf Core-Antigen (nach dem oben angegebenen Enzymimmuntest) und HBsAg (analog dem oben angegebenen Enzymimmuntest) untersucht. Die Fraktion mit der maximalen Core-Antigenaktivität enthält vorwiegend Hepatitis B-Virus-Partikel (HBV-Partikel) und kaum anderes Protein. Die HBsAg Peakfraktion besteht vorwiegend aus HBsAg Filamenten.

## Beispiel III

Monoklonale Antikörper gegen Prä-S(1)

a) Reinigung von Hepatitis B-Viren und Hepatitis B-Oberflächenantigen (HBsAg):

Menschliches Blutplasma (etwa 150 ml) mit mehr als $10^8$ Hepatitis B-Viren pro ml wurde mit TNE-Puffer (0,13 M NaCl, 10 mM Trishydroxymethylaminoethan HCl, pH 7,4, 1 mM EDTA in Wasser) durch eine Chromatographiesäule (10 cm dick, 100 cm lang) mit perlförmigem 6 %igen Agarosegel (zum Beispiel Biogel A5M) gepumpt. Die aufgefangenen 500 Fraktionen von 15 ml wurden auf virales Core Antigen mit dem Enzymimmuntest (J.Virol. Band 42, 1982, Seite 761) untersucht, wobei die Virushülle vor dem Test durch Behandlung mit 0,5 % Nonidet P40 und 0,3 % ß-Mercaptoethanol (jeweils Endkonzentration) vorher abgespalten wurde. Die positiven Fraktionen wurden vereinigt und je 30 ml davon in 10 bis 12 Zentrifugenröhrchen mit 38 ml Fassungsvermögen auf 8 ml 30 %ige Saccharoselösung in TNE-Puffer (Gewicht/Gewicht) geschichtet. Die Röhrchen wurden im Schwingbecherrotor (zum Beispiel SW28 der Firma Beckmann) 24 Stunden bei 25 000 U/min und 10° C zentrifugiert. Der Überstand wurde vollständig entfernt, das Sediment von je 6 Röhrchen in 1 ml TNE-Puffer suspendiert. Diese Virussuspension wurde auf einen Saccharose-Konzentrationsgradienten von 30 bis 55 % in TNE-Puffer in einem 13 ml Zentrifugenröhrchen (zum Beispiel für den Rotor SW 42 Ti der Firma Beckmann) geschichtet und 20 Stunden bei 34 000 U/min und 10° C zentrifugiert. Es wurden Fraktionen von 400 µl nach Anstechen des Röhrchenbodens gesammelt, 25 µl Anteile der Fraktionen wurden quantitativ auf Core-Antigen (nach dem oben angegebenen Enzymimmuntest) und HBsAg (analog

...

dem oben angegebenen Enzymimmuntest) untersucht. Die Fraktion mit der maximalen Core-Antigenaktivität enthält vorwiegend Hepatitis B-Virus-Partikel (HBV-Partikel) und kaum anderes Protein. Die HBsAg Peakfraktion besteht vorwiegend aus HBsAg Filamenten.

b)    Herstellung des Zellklons Go 1/A 18/7-1

0,5 ml der gemäß a) erhaltenen Fraktionen, die vorwiegend HBV-Partikel enthalten, wurden gegen physiologische NaCl-Lösung dialysiert und mit 0,5 ml komplettem Freund'schen Adjuvans emulgiert und intraperitoneal in eine Maus des Stamms Balb/c injiziert. Nach 4 Wochen wurden 0,5 ml HBV-Partikel in inkomplettem Frend'schen Adjuvans (ohne Mycobakterien) erneut injiziert. Vier Tage später wurde die Milz der Maus entnommen, zerkleinert und nach bekannten Methoden (siehe die Broschüre: Hybridoma Techniques, Verlag Cold Spring Harbor Laboratory, 1980, Cold Spring Harbor, Staat New York, USA) mit Maus Myelom Zellen des Typs P3X68-Ag8.653 (J.Immunol. Band 23 (1980), Seite 1548) fusioniert. Die Hybridzellen wurden nach bekannten Methoden (siehe die zuvor genannte Broschüre Hybridoma Techniques) kloniert (Methode der limitierenden Verdünnung), selektioniert und kultiviert. Um die Zellklone zu identifizieren, die Antikörper gegen Hepatitis B-Virus produzieren, wurde die Zellkulturflüssigkeit (das heißt der Überstand der Klone) in Mikrotiternäpfe gegeben, die wie folgt vorbehandelt waren: HBV-Partikel wurden in 20 mM Natriumphosphat pH 7,4 auf 1:100 verdünnt und jeweils 50 Mikroliter dieser verdünnten HBV-Mischung in die Mikrotiternäpfe gegeben; nach 4 Stunden bei Raumtemperatur wurde die HBV-Mischung entfernt. Anschließend wurden 100 Mikroliter einer 1 %igen Rinderserumalbumin-Lösung in der Pufferlösung PBS (130 mM NaCl, 20 mM Natriumphoshat pH 7,5)

...

für 2 Stunden in die Mikrotiternäpfe gegeben, dann entfernt und die Näpfe 3 mal mit PBS-Pufferlösung gewaschen. Die Bindung der Antikörper wurde nach Zugabe von Peroxidase markiertem anti-Maus Immunglobulin durch eine Farbreaktion sichtbar gemacht (siehe die Broschüre Hybridoma Techniques). Die Klone, wo der Überstand eine kräftige Farbreaktion ergab, wurden in größere Kulturgefäße verbracht und darauf geprüft, ob sie mit Prä-S(1)-Polypeptiden (zum Beispiel mit den Peptiden P39 beziehungsweise GP42) reagieren, jedoch nicht mit dem Polypeptid GP33 (Virology, Band 123, 1982, Seiten 436 - 442). Zwei Klone erfüllten diese Voraussetzungen. Einer davon, der als Klon Go 1 A18/7-1 bezeichnet wird, wurde rekloniert und in größeren Kulturen oder in Mäusen (Balb/c) als Ascites-Tumor vermehrt. Der Klon Go 1 A18/7-1 produziert den Antikörper MA 18-7. Der Antikörper befindet sich in der Zellkulturflüssigkeit oder in 1000fach höherer Konzentration in der Ascites Flüssigkeit. (Die Vermehrung in Mäusen erfolgt nach: Kennett, R.H., McKearn, T.J., Bechtol, K.D., Editors, Monoclonal Antibodies, Hybridomas: a new dimension in biological analyses, Plenum Press New York und London, 1982, Seiten 403-404). Der Klon Go 1 A18/7-1 produziert ein Immunglobulin mit den konstanten Eigenschaften $IgG_1$, kappa, das sich spezifisch an P39/GP42 in HBV-Partikeln, HBsAg Filamenten und 20 nm Partikeln und an Prä-S(1)-Polypeptid bindet. Auch an P39/GP42, das durch Detergentien, Reduktionsmittel und Hitze denaturiert wurde, bindet sich der Antikörper MA18/7. Spaltet man P39/GP42 mit V8 Protease, so bindet sich der Antikörper spezifisch an das Prä-S(1)-Polypeptid mit der Masse 18 000 Dalton.

...

ASTA-Werke Aktiengesellschaft, Chemische Fabrik
Artur-Ladebeck-Straße 128-152, 4800 Bielefeld 14


<u>Immunogene Polypeptidsequenz des Hepatitis B-Virus</u>


Ansprüche:


1. DNA-Fragment aus Hepatitis B-Virus oder Hepatitis
B-Virus-Bestandteilen, welches im wesentlichen
aus der Prä-S(1)-Nucleotidsequenz besteht, welche
dadurch gekennzeichnet ist, daß sie die Folge
von 108 oder 119 Codons darstellt, die mit dem
ersten Startcodon des offenen Ableserahmens beginnt, der das Hepatitis B-Oberflächenantigen
(HBsAg) codiert, und 281 Codons vor dessen Stopcodon endet.


2. Polypeptide, bestehend ausschließlich oder im
wesentlichen aus der Prä-S(1)-Aminosäuresequenz,
die von dem DNA-Fragment gemäß Anspruch 1 codiert
wird.


3. Polypeptide, bestehend ausschließlich oder im
wesentlichen aus der Prä-S(1)-Aminosäuresequenz,
dadurch gekennzeichnet,


. . .

daß diese Prä-S(1)-Aminosäuresequenz mit Methionin beginnt, wobei es sich hierbei um das Methionin handelt, welches durch das erste Methionin-Codon (ATG) nach der Schnittstelle der Hepatitis B-DNA mit dem Enzym BstEII codiert wird, und daß das Ende dieser Prä-S(1)-Aminosäuresequenz Alanin ist, welches durch das erste Alanin-Codon (GCC) vor der Schnittstelle der Hepatitis B-DNA mit dem Enzym EcoRI codiert wird.

4. Polypeptide oder Oligopeptide enthaltend die Prä-S(1)-Aminosäuresequenz nach Anspruch 2 oder 3 beziehungsweise die durch die Prä-S(1)-Nucleotidsequenz gemäß Anspruch 1 codiert wird oder immunogene Teilsequenzen dieser Prä-S(1)-Aminosäuresequenz aus mindestens 6 Aminosäuren, ausgenommen das Protein gemäß Anspruch 27 der Europa-Anmeldung 0 020 251.

5. Polypeptide mit der Aminosäuresequenz Prä-S(1)-X nach einem oder mehreren der vorangegangenen Ansprüche, dadurch gekennzeichnet, daß X die Aminosäuresequenz MetGlnTrp-OH, MetHisTrp-OH, MetHisTrpLeuProArgAlaPheArg-OH, MetGlnTrpLeuProArg AlaPheArg-OH, CysLeuAlaArgPheGlyAspAspGlyGluAsnLeu-OH, AlaSerArgValSerValMetThrValLys-OH, ThrSerAspThrCysSerSer ArgArgArgSerLeuLeuValCysLysArgMetProGlyAlaArgLysPro ValArgAlaArgGlnArgValLeuAlaGlyValGlyAlaGlnPro-OH, MetGlnTrpAsnSerThrThrPheHisGlnThrLeuGlnAspProArgVal ArgGlyLeuTyrPheProAlaGlyGlySerSerSerGlyThrSerArgThr GlyAspProAlaThrAsn-OH oder eine andere Peptidsequenz darstellt, die X äquivalent ist, ausgenommen das Protein gemäß Anspruch 27 der Europa-Anmeldung 0 020 251.

...

6. Polypeptide oder Oligopeptide nach einem oder mehreren der vorangegangenen Ansprüche, enthaltend mindestens die Peptidfragmente PheProAsp HisGlnLeuAspProAla; AsnAsnProAspTrpAspPheAsnPro; ThrAsnArgGlnSerGlyArgProThr; AlaAsnArgGlnSerGlyArg ProThr; ProProProAlaSerThrAsnArgGlnSerGlyArgGlnPro ThrPro; ProProProAlaSerAlaAsnArgGlnSerGlyArgGlnPro ThrPro oder mindestens 6 Aminosäuren aus den vorstehend angegebenen Peptidfragmenten in der dort angegebenen Folge, ausgenommen das Protein gemäß Anspruch 27 der Europa-Anmeldung 0 020 251.

7. Oligopeptide der Formel H-PheProAspHisGlnLeuAspPro Ala-OH; H-AsnAsnProAspTrpAspPheAsnPro-OH; H-ThrAsn ArgGlnSerGlyArgProThr-OH; H-AlaAsnArgGlnSerGlyArg ProThr-OH; H-ProProProAlaSerThrAsnArgGlnSerGlyArg GlnProThrPro-OH oder H-ProProProAlaSerAlaAsnArgGln SerGlyArgGlnProThrPro-OH.

8. Polypeptid nach einem oder mehreren der vorangegangenen Ansprüche der Formel I:
H-MetGlyGlyTrpSerSerLysProArgLysGlyMetGlyThrAsnLeuSer ValProAsnProLeuGlyPhePheProAspHisGlnLeuAspProValPheGly AlaAsnSerAsnAsnProAspTrpAspPheAsnProIleLysAspHisTrpPro AlaAlaAsnGlnValGlyValGlyAlaPheGlyProGlyPheThrProProHis GlyGlyValLeuGlyTrpSerProGlnAlaGlnGlyMetLeuThrProValSer ThrIleProProProAlaSerAlaAsnArgGlnSerGlyArgGlnProThrPro IleSerProProLeuArgAspSerHisProGlnAla-X
oder der Formel II
H-MetGlyGlyTrpSerSerLysProArgLysGlyMetGlyThrAsnLeuSer ValProAsnProLeuGlyPhePheProAspHisGlnLeuAspProAlaPheGly AlaAsnSerAsnAsnProAspTrpAspPheAsnProValLysAspAspTrpPro AlaAlaAsnGlnValGlyValGlyAlaPheGlyProArgLeuThrProProHis GlyGlyIleLeuGlyTrpSerProGluAlaGlnGlyIleLeuThrThrValSer ThrIleProProProAlaSerThrAsnArgGlnSerGlyArgGlnProThrPro IleSerProProLeuArgAspSerHisProGlnAla-X

...

oder der Formel III H-MetGlyGlyTrpSerSerLysProArgGlnGlyMetGlyThrAsnLeuSer
ValProAsnProLeuGlyPhePheProAspHisGlnLeuAspProAlaPheGly
AlaAsnSerAsnAsnProAspTrpAspPheAsnProAsnLysAspGlnTrpPro
GluAlaAsnGlnValGlyAlaGlyAlaPheGlyProGlyPheThrProProHis
GlyGlyLeuLeuGlyTrpSerProGlnAlaGlnGlyIleLeuThrThrValPro
AlaAlaProProProAlaSerThrAsnArgGlnSerGlyArgGlnProThrPro
IleSerProProLeuArgAspSerHisProGlnAla-X

oder der Formel IV H-MetGlyGlnAsnLeuSerThrSerAsnProLeuGlyPhePheProAspHis
GlnLeuAspProAlaPheArgAlaAsnThrAlaAsnProAspTrpAspPheAsn
ProAsnLysAspThrTrpProAspAlaAsnLysValGlyAlaGlyAlaPheGly
LeuGlyPheThrProProHisGlyGlyLeuLeuGlyTrpSerProGlnAlaGln
GlyIleLeuGluThrLeuProAlaAsnProProProAlaSerThrAsnArgGln
SerGlyArgGlnProThrProLeuSerProProLeuArgAsnThrHisProGln
Ala-X worin X=OH oder die Peptidfragmente MetGlnTrp-OH,
MetHisTrp-OH, MetHisTrpLeuProArgAlaPheArg-OH,
MetGlnTrpLeuProArgAlaPheArg-OH, CysLeuAlaArgPheGly
AspAspGlyGluAsnLeu-OH, AlaSerArgValSerValMetThrVal
LysThrSerAspThrCysSerSerArgArgArgSerLeuValCysLysArg
MetProGlyAlaArgLysProValArgAlaArgGlnArgValLeuAlaGly
ValGlyAlaGlnPro-OH oder die Prä-S(2)-Sequenz MetGln
TrpAsnSerThrAlaPheHisGlnAlaLeuGlnAspProArgValArgGly
LeuTyrPheProAlaGlyGlySerSerSerGlyThrValAsnProAlaPro
AsnIleAlaSerHisIleSerSerIleSerAlaArgThrGlyAspProVal
ThrAsn-OH darstellt und die äquivalenten Varianten
dieser Peptide.

9. Recombiniertes DNA-Molekül, welches zur Expression
der Prä-S(1)-Sequenz in Bakterien, Hefe oder
tierischen Zellen befähigt ist.

...

10. DNA-Fragment, welches mindestens die Prä-S(1)-Nucleotidsequenz, die in Fig. 1 dargestellt ist, oder Untereinheiten dieser Nucleotidsequenz aus mindestens 6 Codons enthält.

11. DNA-Vektor vom Typ der Plasmide oder Phagen, enthaltend ein recombiniertes DNA-Molekül nach einem oder mehreren der vorangegangenen Ansprüche zur Expression der gesamten Prä-S(1)-Aminosäuresequenz oder von Teilen dieser Prä-S(1)-Aminosäuresequenz aus mindestens 6 Aminosäuren.

12. DNA-Vektor nach einem oder mehreren der vorangegangenen Ansprüche, der das AluI/EcoRI-Fragment oder das BstEII + Bal31/EcoRI-Fragment oder das BglII/EcoRI-Fragment der Hepatitis B-DNA mit der Prä-S(1)-Nucleotidsequenz enthält.

13. Expressionsvektoren nach einem oder mehreren der vorangegangenen Ansprüche und deren Verwendung zur Herstellung eines Proteins, welches als Impfstoff gegen Hepatitis B-Infektionen verwendbar ist.

14. Escherichia coli K12 Abkömmlinge, welche Prä-S(1)-Polypeptide exprimieren wie hinterlegt bei "Deutsche Sammlung von Mikroorganismen (DSM), Griesebachstraße 8, D-3400 Göttingen" mit der Hinterlegungsnummer DSM 3079, DSM 3080 und DSM 3081.

...

15. Verfahren zur Herstellung von Polypeptiden, die durch die Prä-S(1)-Nucleotidsequenz der Hepatitis B-Virus DNA codiert werden beziehungsweise zur Herstellung von Polypeptiden, die die Prä-S(1)-Aminosäuresequenz oder Untereinheiten aus mindestens 6 Aminosäuren hiervon enthalten, dadurch gekennzeichnet, daß man in an sich bekannter Weise

a) Hepatitis B-Virus DNA gleichzeitig oder nacheinander

1) mit Restriktionsendonucleasen behandelt, die die Nucleotidsequenz GAATTC spalten,

2) mit Restriktionsendonucleasen behandelt, die die Nucleotidsequenz AGCT und/oder die Nucleotidsequenz AGATCT und/oder die Nucleotidsequenz GGTNACC spalten

und die so erhaltenen DNA-Fragmente, die im wesentlichen aus der Prä-S(1)-Sequenz bestehen, mit der DNA eines üblichen Vektors rekombiniert, die rekombinierte Vektor-DNA in einen bakteriellen Wirtsorganismus einführt und nach Selektion und Klonierung die Polypeptide mit der Prä-S(1)-Aminosäuresequenz zur Expression bringt und isoliert

oder

b) Hepatitis B-Oberflächenantigen oder Hepatitis B-Virus-Partikel mit einem ionischen Detergenz in Gegenwart von Disulfidbrücken spaltenden Reagenzien in Einzelproteine zerlegt und diese mit Bromcyan oder mit einer Glutaminsäure spezifischen Protease behandelt, gegebenenfalls anschließend noch mit einer Glutaminsäure spezifischen Protease behandelt und die Proteinfragmente, die die Prä-S(1)-

...

Sequenz oder gegebenenfalls Untereinheiten der Prä-S(1)-Sequenz enthalten, isoliert

oder

c) Peptide gemäß Anspruch 7 oder 8 aus den entsprechenden Aminosäuren synthetisiert, indem man zunächst die Carboxy-terminale Aminosäure des zu synthetisierenden Peptids, deren $\alpha$-ständige Aminogruppe geschützt ist, an einen hierfür üblichen synthetischen Träger kovalent bindet, die $\alpha$-Amino-Schutzgruppe abspaltet, an die so erhaltene freie Aminogruppe die nächstfolgende geschützte Aminosäure über ihre Carboxy-Gruppe bindet, dann wiederum die $\alpha$-Amino-Schutzgruppe dieser zweiten Aminosäure abspaltet, hieran die nächste Aminosäure bindet und in dieser Weise Schritt für Schritt die übrigen Aminosäuren des zu synthetisierenden Peptids in der richtigen Reihenfolge verknüpft und nach Verknüpfung aller Aminosäuren das fertige Peptid vom Träger abspaltet und gegebenenfalls weitere vorhandene Seitenfunktions-Schutzgruppen abspaltet.

16. Polypeptide mit der Prä-S(1)-Aminosäuresequenz oder Untereinheiten der Prä-S(1)-Aminosäuresequenz aus mindestens 6 Aminosäuren erhältlich nach Anspruch 15.

17. Verwendung von Polypeptiden mit der Prä-S(1)-Aminosäuresequenz oder Untereinheiten dieser Sequenz aus mindestens 6 Aminosäuren nach einem oder mehreren der vorangegangenen Ansprüche zur Herstellung eines Impfstoffes gegen Hepatitis B-Erkrankungen.

...

18. Impfstoff gegen Hepatitis B-Virus-Erkrankungen
dadurch gekennzeichnet,
daß er als Wirkstoff mindestens ein Peptid mit
der Prä-S(1)-Aminosäuresequenz oder Untereinheiten
dieser Sequenz aus mindestens 6 Aminosäuren nach
einem oder mehreren der vorangegangenen Ansprüche
enthält.

19. Impfstoff gegen Hepatitis B-Virus-Erkrankungen
dadurch gekennzeichnet,
daß er als Wirkstoff mindestens ein Peptid mit
der Prä-S(1)-Aminosäuresequenz oder Untereinheiten
dieser Sequenz aus mindestens 6 Aminosäuren nach
einem oder mehreren der vorangegangenen Ansprüche
sowie pharmazeutisch verträgliche Träger-, Zusatz-
und/oder Hilfsstoffe und/oder Adjuvanzien enthält.

20. Verfahren zur Herstellung eines Arzneimittels
gegen Hepatitis B-Virus-Erkrankungen,
dadurch gekennzeichnet,
daß ein Peptid mit der Prä-S(1)-Aminosäuresequenz
oder Untereinheiten dieser Sequenz aus mindestens
6 Aminosäuren nach einem oder mehreren der vorangegangenen Ansprüche mit Adjuvanzien oder sonstigen
pharmazeutisch verträglichen Trägern oder Hilfsstoffen zu einem Impfstoff verarbeitet wird.

21. Monoklonaler Antikörper MA18/7, der Hepatitis B-
Virus Peptidstrukturen neutralisiert, die für die
Induktion einer gruppenspezifischen Immunität
verantwortlich sind.

...

22. Maus-Hybridoma-Zellen mit der Bezeichnung Go 1 A 18/7-1 die den Antikörper MA18/7 synthetisieren wie hinterlegt bei Public Health Laboratory Service, Porton Down, Salisbury, Wiltshire SP40JG, Großbritannien unter der Hinterlegungsnummer 84101501.

23. Verfahren zur Herstellung des monoklonalen Antikörpers MA18/7,
dadurch gekennzeichnet,
daß man Milzzellen von gegen Hepatitis B-Virus immunisierten Mäusen mit Myelomzellen fusioniert, von den erhaltenen Hybridzellklonen diejenigen auswählt, die nur mit Hepatitis B-Virus Peptiden, welche die Prä-S(1)-Aminosäuresequenz enthalten, reagieren und die so ausgewählten Klone vermehrt und den Antikörper aus der so erhaltenen Kulturflüssigkeit isoliert.

24. Verwendung des monoklonalen Antikörpers MA18/7 in der Diagnose und Therapie von Hepatitis B-Virus-Erkrankungen.

...

Fig. 1

Prä-S(1) Sequenz von pHBV991 und Sequenz des exprimierten Polypeptids

```
Met Gly Gly Trp Ser Ser Lys Pro Arg Lys Gly Met Gly Thr Asn Leu Ser Val Pro Asn Pro Leu Gly Phe Phe    25
ATG GGA GGT TGG TCA TCA AAA CCT CGC AAA GGC ATG GGG ACG AAT CTT TCT GTT CCC AAC CCT CTG GGA TTC TTT
Start
Prä-S(1)

Pro Asp His Gln Leu Asp Pro Val Phe Gly Ala Asn Ser Asn Asn Pro Asp Trp Asp Phe Asn Pro Ile Lys Asp    50
CCC GAT CAT CAG TTG GAC CCT GTA TTC GGA GCC AAC TCA AAC AAT CCA GAT TGG GAC TTC AAC CCC ATC AAG GAC
            Sau 96I

His Trp Pro Ala Ala Asn Gln Val Gly Val Gly Ala Phe Gly Pro Gly Phe Thr Pro Pro His Gly Gly Val Leu    75
CAC TGG CCA GCA GCC AAC CAG GTA GGA GTG GGA GCA TTC GGG CCA GGG TTC ACC CCT CCA CAC GGC GGT GTT TTG
    BalI

Gly Trp Ser Pro Gln Ala Gln Gly Met Leu Thr Pro Val Ser Thr Ile Pro Pro Pro Ala Ser Ala Asn Arg Gln    100
GGG TGG AGC CCT CAG GCT CAG GGC ATG TTG ACC CCA GTG TCA ACA ATT CCT CCT CCT GCC TCC GCC AAT CGG CAG

Ser Gly Arg Gln Pro Thr Pro Ile Ser Pro Pro Leu Arg Asp Ser His Pro Gln Ala Met Gln Trp    122
TCA GGA AGG CAG CCT ACT CCC ATC TCT CCA CCT CTA AGA GAC AGT CAT CCT CAG CCC ATG CAG TGG
                                                                      Start
                                                                      Prä-S(2)
```

**Fig. 2**

```
TTACATACTCTTTGGAAGGCTGGTATTCTATATAAGCGGGAAACCACACGTAGCGCATCATTTTGCGGGTCACCATATTCTTGGGAACAAGAGCTACAGC          2853
                                                                       BstEII                    AluI
```

```
Met Gly Gly Trp Ser Ser Lys Pro Arg Lys Gly Met Gly Thr Asn Leu Ser Val Pro Asn Pro Leu Gly Phe Phe
ATG GGA GGT TGG TCA TCA AAA CCT CGC AAA GGC ATG GGG ACG AAT CTT TCT GTT CCC AAT CCT CTG GGA TTC TTT          2928
Start
Prae-S(1)
```

```
Pro Asp His Gln Leu Asp Pro Ala Phe Gly Ala Asn Ser Asn Asn Pro Asp Trp Asp Phe Asn Pro Val Lys Asp
CCC GAT CAT CAG TTG GAC CCT GCA TTC GGA GCC AAC TCA AAC AAT CCA GAT TGG GAC TTC AAC CCC GTC AAG GAC          3003
                  Sau 96I
```

```
Asp Trp Pro Ala Ala Asn Gln Val Gly Val Gly Ala Phe Gly Pro Arg Leu Thr Pro Pro His Gly Gly Ile Leu
GAC TGG CCA GCA GCC AAC CAA GTA GGA GTG GGA GCA TTC GGG CCA AGG CTC ACC CCT CCA CAC GGC GGT ATT TTG          3078
    BalI
```

```
Gly Trp Ser Pro Glu Ala Gln Gly Ile Leu Thr Thr Val Ser Thr Ile Pro Pro Pro Ala Ser Thr Asn Arg Gln
GGG TGG AGC CCT CAG GCT CAG GGC ATA TTG ACC ACA GTG TCA ACA ATT CCT CCT CCT GCC TCC ACC AAT CGG CAG          3153
```

```
Ser Gly Arg Gln Pro Thr Pro Ile Ser Pro Pro Leu Arg Asp Ser His Pro Gln Ala Met Gln Trp Asn Ser Thr
TCA GGA AGG CAG CCT ACT CCC ATC TCT CCA CCT CTA AGA GAC AGT CAT CCT CAG GCC ATG CAG TGG AAT TCC ACT
                                                                       Start       EcoRI
                                                                       Prae-S(2)
```

**Fig.3**

TTACATACTCTGTGGAAGGCTGGCATTCTATATAAGAGAGAAACTACACGCAGCGCCTCATTTTGTGGGTCACCATATTCTTGGGAACAAGAGCTACAGC     2853
<br>                                                                BstEII                                AluI

Met Gly Gly Trp Ser Ser Lys Pro Arg Gln Gly Met Gly Thr Asn Leu Ser Val Pro Asn Pro Leu Gly Phe Phe
<br>ATG GGA GGT TGG TCT TCC AAA CCT CGA CAA GGC ATG GGG ACG AAT CTT TCT GTT CCC AAT CCT CTG GGA TTC TTT     2928
<br>Start Prae-S(1)               TaqI

Pro Asp His Gln Leu Asp Pro Ala Phe Gly Ala Asn Ser Asn Asn Pro Asp Trp Asp Phe Asn Pro Asn Lys Asp
<br>CCC GAT CAC CAG TTG GAC CCT GCG TTC GGA GCC AAC TCA AAC AAT CCA GAT TGG GAC TTC AAC CCC AAC AAG GAT     3003
<br>                  Sau 96I

Gln Trp Pro Glu Ala Asn Gln Val Gly Ala Gly Ala Phe Gly Pro Gly Phe Thr Pro Pro His Gly Gly Leu Leu
<br>CAA TGG CCA GAG GCA AAT CAG GTA GGA GCG GGA GCA TTC GGG CCA GGG TTC ACC CCA CCA CAC GGC GGT CTT TTG     3078
<br>    BalI

Gly Trp Ser Pro Gln Ala Gln Gly Ile Leu Thr Thr Val Pro Ala Ala Pro Pro Pro Ala Ser Thr Asn Arg Gln
<br>GGG TGG AGC CCT CAG GCT CAG GGC ATA TTG ACA ACA GTG CCA GCA GCA CCT CCT CCT GCC TCC ACC AAT CGG CAG     3153

Ser Gly Arg Gln Pro Thr Pro Ile Ser Pro Pro Leu Arg Asp Ser His Pro Gln Ala Met Gln Trp Asn Ser Thr
<br>TCA GGA AGA CAG CCT ACT CCC ATC TCT CCA CCT CTA AGA GAC AGT CAT CCT CAG GCC ATG CAG TGG AAT TCC ACA     7
<br>                                                                Start        EcoRI
<br>                                                                Prae-S(2)

*Fig. 4*

TTACACACTCTATGGAAGGAGGGTATATTATATAAGAGAGAAACAACACATAGCGCCTCATTTTGTGGGTCACCATATTCTTGGGAACAAGATCTACAGC    2853
                                                                           BstEII

Met Gly Gln Asn Leu Ser Thr Ser Asn Pro Leu Gly Phe Phe
------------------Deletion ------------------ATG GGG CAG AAT CTT TCC ACC AGC AAT CCT CTG GGA TTC TTT    2928
                                              Start
                                              Prae-S(1)

Pro Asp His Gln Leu Asp Pro Ala Phe Arg Ala Asn Thr Ala Asn Pro Asp Trp Asp Phe Asn Pro Asn Lys Asp
CCC GAT CAC CAG TTG GAT CCA GCC TTC AGA GCA AAC ACC GCA AAT CCA GAT TGG GAC TTC AAT CCC AAC AAG GAC    3003

Thr Trp Pro Asp Ala Asn Lys Val Gly Ala Gly Ala Phe Gly Leu Gly Phe Thr Pro Pro His Gly Gly Leu Leu
ACC TGG CCA GAC GCC AAC AAG GTA GGA GCT GGA GCA TTC GGG CTG GGT TTC ACC CCA CCG CAC GGA GGC CTT TTG    3078
    BalI

Gly Trp Ser Pro Gln Ala Gln Gly Ile Leu Glu Thr Leu Pro Ala Asn Pro Pro Pro Ala Ser Thr Asn Arg Gln
GGG TGG AGC CCT CAG GCT CAG GGC ATA CTA CAA ACT TTG CCA GCA AAT CCG CCT CCT GCC TCC ACC AAT CGC CAG    3153

Ser Gly Arg Gln Pro Thr Pro Leu Ser Pro Pro Leu Arg Asn Thr His Pro Gln Ala Met Gln Trp Asn Ser Thr
TCA GGA AGG CAG CCT ACC CCG CTG TCT CCA CCT TTG AGA AAC ACT CAT CCT CAG GCC ATG CAG TGG AAT TCC ACT    7
                                                                      Start        EcoRI
                                                                      Prae-S(2)

<div align="center">Fig. 5</div>

16 15 14 13 12 11 10 9 8 7 6 5 4 3 2 1    Kupplungs-
Methode

Pro-Pro-Pro-Ala-Ser-Thr/Ala-Asn-Arg-Gln-Ser-Gly-Arg-Gln-Pro-Thr-Pro

BOC-Pro - R                                    SA

BOC-Thr(O-Bzl)                                 SA

BOC-Pro                                         DCC/HOBT

BOC-Gln                                         DCC

BOC-Arg(NO$_2$)                                 SA

BOC-Gly                                         SA

BOC-Ser(O-Bzl)                                  DCC/HOBT

BOC-Gln                                         DCC

BOC-Arg(NO$_2$)                                 DCC/HOBT

BOC-Asn                                         SA

BOC-Thr(O-Bzl)(bzw.BOC-Ala)                     SA

BOC-Ser(O-Bzl.)                                 SA

BOC-Ala                                         SA

BOC-Pro                                         SA

BOC-Pro                                         SA

BOC-Pro                                         SA

*Fig. 6*

Festphasen-Peptidsynthese-Plan:  R = PAM-Polystyrol; BOC = N-Butyloxycarbonyl;
SA = Symmetrische Anhydride; DCC/HOBT = Dicyclohexylcarbodiimid/1-Hydroxybenzotriazol,
DCC = Dicyclohexylcarbodiimid.

0180012

**Europäisches Patentamt**

**EUROPÄISCHER RECHERCHENBERICHT**

| | EINSCHLÄGIGE DOKUMENTE | | EP 85111361.3 |
|---|---|---|---|

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A,P | EP - A2 - 0 154 902 (NEW YORK BLOOD CENTER, INC.; CALIFORNIA INSTITUTE OF TECHNOLOGY)<br><br>* Ansprüche 1,2,7; Seite 4 *<br><br>-- | 1,2,10, 17-20 | C 12 N 15/00<br>C 07 H 21/04<br>C 07 K 15/00<br>C 12 P 21/00<br>C 12 N 1/20 |
| D,A | EP - A2 - 0 020 251 (THE REGENTS OF THE UNIVERSITY OF CALIFORNIA)<br><br>* Ansprüche 11-24,27,28; Tabelle 2 *<br><br>-- | 1-4,7-11,13, 15-20 | A 61 K 39/29<br>A 61 K 39/42<br>C 12 N 5/00<br>G 01 N 33/576 |
| A | SCIENCE, Vol. 213, No. 4506, 24. Juli 1981 (Washington D.C.)<br><br>P. TIOLLAIS et al. "Biology of Hepatits B Virus" Seiten 406-411<br><br>* Gesamt *<br><br>---- | 1 | //C 12 R 1:19 |

| RECHERCHIERTE SACHGEBIETE (Int. Cl.4) |
|---|
| C 12 N |
| C 07 H |
| C 07 K |
| C 12 P |
| A 61 K |
| G 01 N |

Der vorliegende Recherchenbericht wurde für alle Patentansprüche erstellt.

| Recherchenort | Abschlußdatum der Recherche | Prüfer |
|---|---|---|
| WIEN | 20-01-1986 | WOLF |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82